# EUROPEAN PATENT SPECIFICATION

(11) **EP 0 932 609 B1**
(45) Date of publication and mention of the grant of the patent: **14.05.2003**
(21) Application number: 97910866.9
(22) Date of filing: 10.10.1997
(51) Int. Cl.: C07D 491/056, A61K 31/40

(54) **AZAHETEROCYCLYMETHYL DERIVATIVES OF 2,3,8,9-TETRAHYDRO-7H-1,4-DIOXINO 2,3-e]INDOL-8-ONE**
AZAHETEROCYCLYLMETHYL-DERIVATE VON 2,3,8,9-TETRAHYDRO-7H-1,4-DIOXINO[2,3-E]INDOL-8-ON
DERIVES DE 2,3,8,9-TETRAHYDRO-7H-1,4-DIOXINO2.3-EINDOL-8-ONE DE AZAHETEROCYCLYLMETHYLE

(30) Priority: 15.10.1996 US 732807
(43) Date of publication of application: 04.08.1999
(73) Proprietor: Wyeth, Madison, New Jersey 07940-0874 (US)
(72) Inventor: STACK, Gary, Paul, Ambler, PA 19002 (US)
(74) Representative: Mannion, Sally Kim, Dr.
(86) International application number: US9718275
(87) International publication number: WO98016530

(56) References cited:
- EP-A- 0 771 801
- WO-A-91/13872
- US-A- 5 318 988
- US-A- 5 371 094

## Description

### Background of the Invention

PCT Int. Appl. WO 91 13,872 discloses dioxino[2,3-e]indole derivatives of the formula I, in which R¹ is H, alkyl, CO₂R², CONHR², cyano, halo, CHO, etc.; R² is H, alkyl, (CH₂)ₘY; Y is cycloalkyl or cycloalkenyl, (substituted)phenyl, pyridyl, naphthyl, indolyl; m is 0-6; A and B are O, CH₂, S; and X is defined by formulas a, b or c, in which R² and m are defined as above, R³ is hydrogen, -CO₂R², -CONHR², -CN, -NHR², -CHO, -((CH₂)ₘ-Ar, -NR²Ar or 1-benzimidazol-2-one, and R⁴ is hydrogen, C₁-C₆ alkyl, C₂-C₈ alkenyl, C₂-C₈ alkynyl, -(CH₂)ₘ-(C₃-C₈) cycloalkyl or cycloalkenyl, -(CH₂)ₘ -Ar, - CO₂R², - CONHR², -CN or -CHO, as serotonergic and dopaminergic agents useful for the treatment of CNS and cardiovascular disorders.

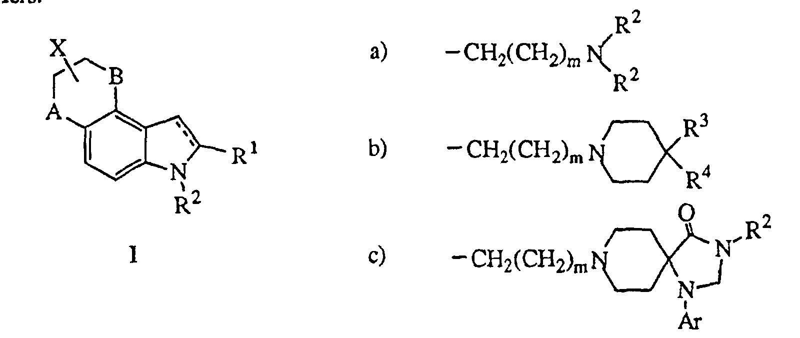

US 5,318,988 discloses 2-aminomethyl-chromans of formula II as useful for treatment of diseases of the central nervous system. In this group of compounds, A, B and D are identical or different and represent hydrogen, halogen, cyano, azido, nitro, di- or tri-fluoromethyl, di- or tri-fluoromethoxy, hydroxyl or carboxyl, straight-chain or branched-chain alkyl, alkenyl, acyl, alkoxy or alkoxycarbonyl, or a mono- or di-substituted or unsubstituted amino, amido or sulfonamido, or A may be so defined and B and D taken together to form a 5 to 7-membered saturated, partly unsaturated, or aromatic carbocyclic ring or heterocyclic ring 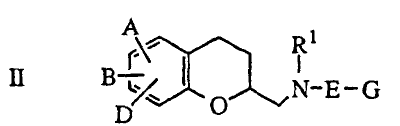 having up to two S, N or O atoms, optionally one or two carbonyl functions in the ring and optionally ring substituted by alkyl, branched alkyl or cycloalkyl; E represents a direct bond or represents straight chain or branched chain alkylene, alkenylene or alkynylene; G represents aryl having 6 to 10 carbon atoms or a 5 to 7-membered, saturated or unsaturated heterocyclic ring which is not bonded via N and has up to 3 hetero atoms from the series comprising N, O or S, to which a further saturated, partly unsaturated or aromatic 6-membered ring can optionally also be fused or cycloalkyl or a bridged bicarbocyclic ring. US 5,371,094, related to the above, replaces NR¹-E-G in formula II with substituted piperidine, substituted tetrahypyridine or substituted dihydroisoindole and claims utility in the treatment of anxiety.

### Description of the Invention

In accordance with this invention, there is provided a group of novel antipsychotic agents of formula I: 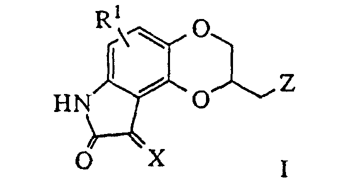 wherein
X is H₂ or O;
R¹ is hydrogen, hydroxy, halo, trifluoromethyl, trifluoromethoxy, alkyl of 1 to 6 carbon atoms, alkoxy of 1 to 6 carbon atoms, aralkoxy of 7 to 12 carbon atoms, alkanoyloxy of 2 to 6 carbon atoms, amino, mono- or dialkylamino in which each alkyl group has 1 to 6 carbon atoms, alkanamido of 2 to 6 carbon atoms or alkanesulfonamido of 1 to 6 carbon atoms;
Z is 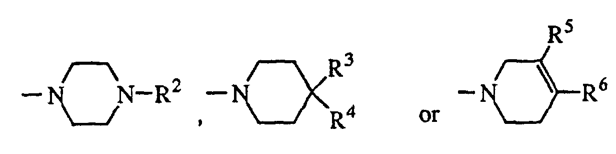 wherein
R² is hydrogen, alkyl of 1 to 6 carbon atoms, cycloalkyl of 3 to 8 carbon atoms, phenyl optionally substituted with R¹ as defined above; ω-phenylalkyl or ω-diphenylalkyl, in which the alkyl chain contains 1 to 4 carbon atoms and the phenyl is optionally substituted with R¹ as defined above, or R² is naphthyl, indolyl, indazolyl, thienyl, pyridinyl, pyrimidinyl, quinolinyl, benzoisothiazolyl or benzisoxazolyl, each optionally substituted with R¹ as defined above;
R³ is hydrogen and R⁴ is hydrogen, 1-benzimidazolyl-2-one, indolyl, benzoisothiazolyl or benzisoxazolyl, each optionally substituted with R¹ as defined above, or R⁴ is -Y-Ar, in which Y is C=O, CHOH, or (CH₂)ₘ, wherein m is 0 to 4, and Ar is phenyl, optionally substituted with R¹ as defined above,
or R³ and R⁴, taken together with the carbon atom to which they are attached form 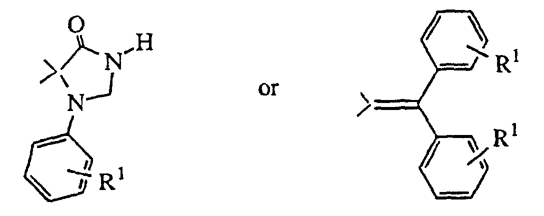
R⁵ is hydrogen and R⁶ is phenyl, naphthyl, thienyl, indolyl, benzoisothiazolyl or benzisoxazolyl, each optionally substituted with R¹ as defined above,
or R⁵ and R⁶, taken together with the carbon atoms to which they are attached complete a benzene ring optionally substituted with R¹;
or a pharmaceutically acceptable salt thereof.

Of these compounds, the preferred members are those in which X and R¹ are defined as above, R² is phenyl, naphthyl, indolyl, indazolyl, thienyl, pyridinyl, pyrimidinyl, quinolinyl, benzoisothiazolyl or benzisoxazolyl, each optionally substituted with R¹ as dcfined above, R³ is hydrogen and R⁴ is 1-benzimidazolyl-2-one, indolyl, benzoisothiazolyl or benzisoxazolyl, each optionally substituted with R¹ as defined above, or R⁴ is -Y-Ar, in which Y is C=O, and Ar is phenyl, optionally substituted with R¹ as defined above, R⁵ is hydrogen and R⁶ is phenyl, indolyl, benzoisothiazolyl or benzisoxazolyl, each optionally substituted with R¹ as defined above, or R⁵ and R⁶, taken together with the carbon atoms to which they are attached complete a benzene ring optionally substituted with R¹, defined above.

When either of R¹ or R² is alkyl of 1 to 6 carbon atoms or the R¹ or R² group contains alkyl, the alkyl group preferably contains 1 to 4 atoms carbon atoms, and is suitably methyl, ethyl, n-propyl, iso-propyl, n-butyl, iso-butyl or tert-butyl. When R¹ is halogen it is preferably fluorine or chlorine, particularly fluorine. R¹ is preferably hydrogen or halogen.

Most preferred are those members in which X is H₂ and R¹ is defined as above, R² is phenyl, indolyl, pyridinyl, pyrimidinyl, quinolinyl, benzoisothiazolyl or benzisoxazolyl, each optionally substituted with R¹ as defined above, R³ is hydrogen and R⁴ is 1-benzimidazolyl-2-one, indolyl, benzoisothiazolyl or benzisoxazolyl, each optionally substituted with R¹ as defined above, or R⁴ is -Y-Ar, in which Y is C=O, and Ar is phenyl, optionally substituted with R¹ as defined above,. R⁵ is hydrogen and R⁶ is phenyl, optionally substituted with R¹ as defined above, or R⁵ and R⁶, taken together with the carbon atoms to which they are attached complete a benzene ring optionally substituted with R¹, as defined above. This invention relates to both the R and S stereoisomers of the aminomethyl-2,3,8,9-tetrahydro-7H-1,4-dioxino[2,3-e]indol-8-ones, as well as to mixtures of the R and S stereoisomers. Throughout this application, the name of the product of this invention, where the absolute configuration of the aminomethyl-2,3,8,9-tetrahydro-7H-1,4-dioxino[2,3-e]indol-8-ones is not indicated, is intended to embrace the individual R and S enantiomers as well as mixtures of the two.

The pharmaceutically acceptable salts are those derived from such organic and inorganic acids as: acetic, lactic, citric, tartaric, succinic, fumaric, maleic, malonic, mandelic, mallic, hydrochloric, hydrobromic, phosphoric, nitric, sulfuric, methanesulfonic, toluenesulfonic and similarly known acceptable acids.

The 2-azaheterocyclylmethyl-2,3,8,9-tetrahydro-7H-1,4-dioxino[2,3-e]indol-8-ones are prepared as illustrated below for examples in which Z is substituted piperazine. Specifically, the appropriately substituted nitroguaiacol is alkylated with allyl bromide in the presence of a suitable base such as sodium hydride and then 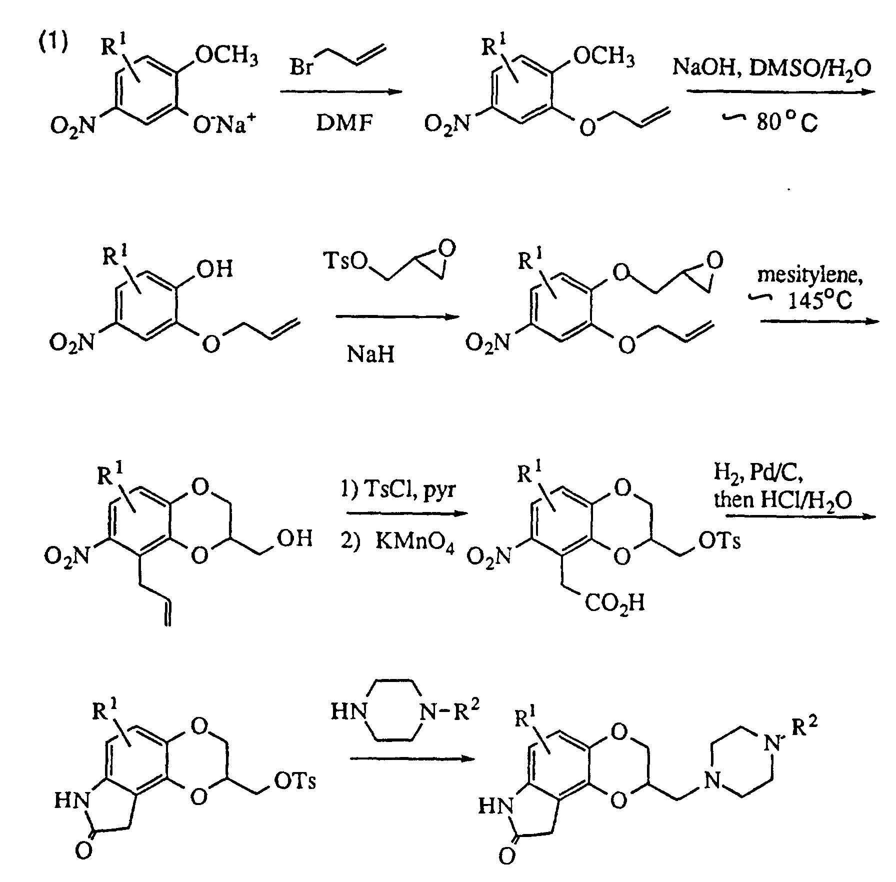 demethylated by a reagent such as sodium hydroxide. The resulting 4-nitro-2-allyloxyphenol is then alkylated with glycidyl tosylate or an epihalohydrin in the presence of a base such as sodium hydride and heated in a high boiling solvent such as mesitylene or xylene to effect both rearrangement of the allyl group and cyclization of the dioxan ring. The resulting primary alcohol is converted to the tosylate by reaction with p-toluenesulfonyl chloride in the presence of pyridine or alternatively to a halide by reaction with carbon tetrabromide or carbon tetrachloride in combination with triphenylphosphine. The allyl side chain is converted to an acetic acid moiety by oxidative cleavage with potassium permanganate and the nitro group is reduced to an amine with hydrogen and palladium on carbon and cyclized to the lactam with aqueous hydrochloric acid. Replacement of the tosylate or halide with the appropriately substituted azaheterocycle in some high boiling solvent such as dimethyl sulfoxide gives the title compounds of the invention.

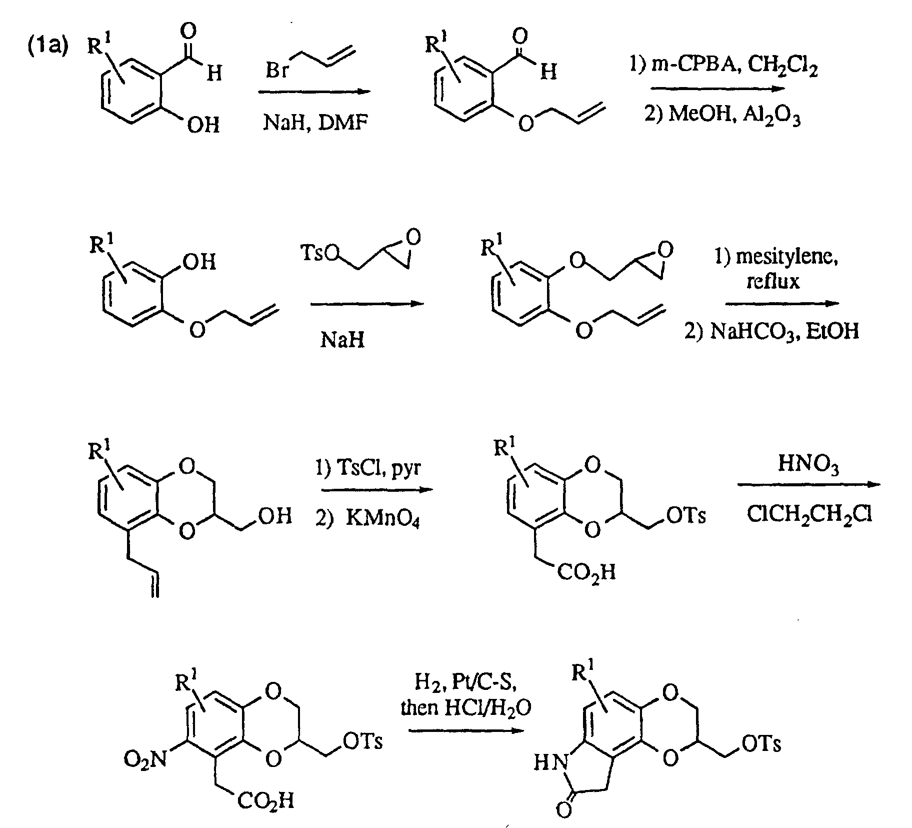

The oxindoledioxan methyltosylate described in (1) may also be prepared as in (1a) above: the appropriately substituted salicylaldehyde is alkylated with allyl bromide in the presence of a suitable base such as sodium hydride. The aldehyde moiety is then converted to a phenol by treatment with m-chloroperoxybenzoic acid followed by cleavage of the intermediate formate ester with basic alumina in methanol. The resulting 2-allyloxyphenol is then alkylated with glycidyl tosylate or an epihalohydrin in the presence of a base such as sodium hydride and heated in a high boiling solvent such as mesitylene or xylene to effect rearrangement of the allyl group. Cyclization to the benzodioxanmethanol is completed by treatment with sodium bicarbonate in ethanol. Following conversion of the alcohol to a tosylate via p-toluenesulfonyl chloride in pyridine, the allyl side chain is oxidatively cleaved to an acetic acid moiety with potassium permanganate and the nitro group introduced by treatment with nitric acid in dichloroethane. Reduction of the nitro group and cyclization to the lactam are effected as in (1). A catalyst such as platinum oxide or platinum on sulfided carbon is preferred for the reduction when R¹ is a halogen.

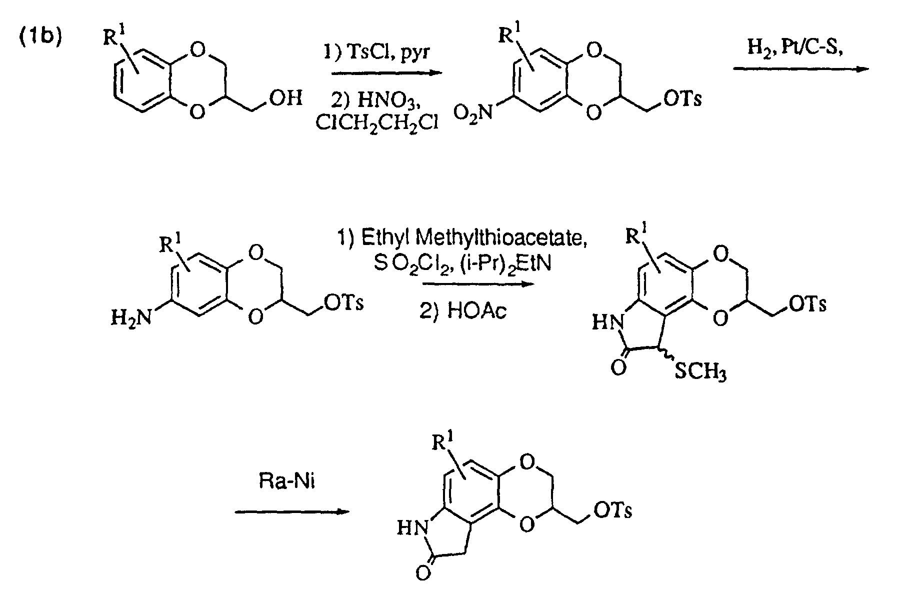

The oxindoledioxan methyltosylate may also be prepared from the appropriately substituted benzodioxan methanol as in (1b) above. Following conversion of the alcohol to the tosylate as described above, the nitro function is introduced by treatment with nitric acid in dichloroethane and reduced with hydrogen in the presence of a suitable catalyst such as platinum oxide or platinum on sulfided carbon. The oxindole is elaborated by a modification of the procedure of Gassman *et. al.* [*J.* *Amer. Chem. Soc*. 96, 5512 (1974)] and the resulting thiomethyl ether cleaved by treatment with Raney nickel.

Compounds of the invention in which X is oxygen (i.e., isatins) may be prepared by oxidation of the corresponding oxindoles. The appropriate nitroguaiacols are known compounds or may be prepared by one schooled in the art. Alternatively, the 4-nitro-2-allyloxyphenols utilized in process (1) described above may be prepared from the appropriately 5- or 6-substituted salicylaldehyde by procedure (2) below, or from the appropriately 3- or 4-substituted salicylaldehyde by procedure (3) below , in which [2-(trimethylsilyl)ethoxy]methyl chloride (SEMCI) is employed as a hydroxy protecting group during conversion of the aldehyde to the formate ester with metachloroperbenzoic acid followed by hydrolysis to the hydroxy group. The substituted azaheterocycles are known compounds or may be readily prepared by one schooled in the art. The compounds of the invention may be resolved into their enantiomers by conventional methods or, preferably, they may be prepared directly by substitution of (2R)-(-)-glycidyl 3-nitrobenzenesulfonate or tosylate (for the S benzodioxan methanamine) or (2S)-(+)-glycidyl 3-nitrobenzenesulfonate or tosylate (for the R enantiomer) in place of epihalohydrin or racemic glycidyl tosylate in the procedures above.

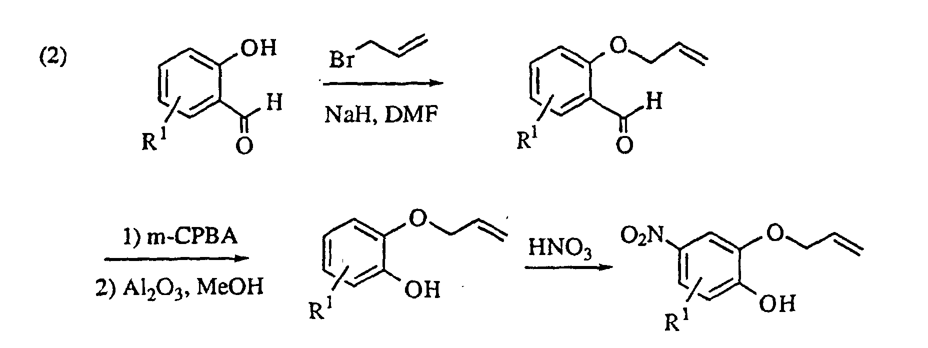

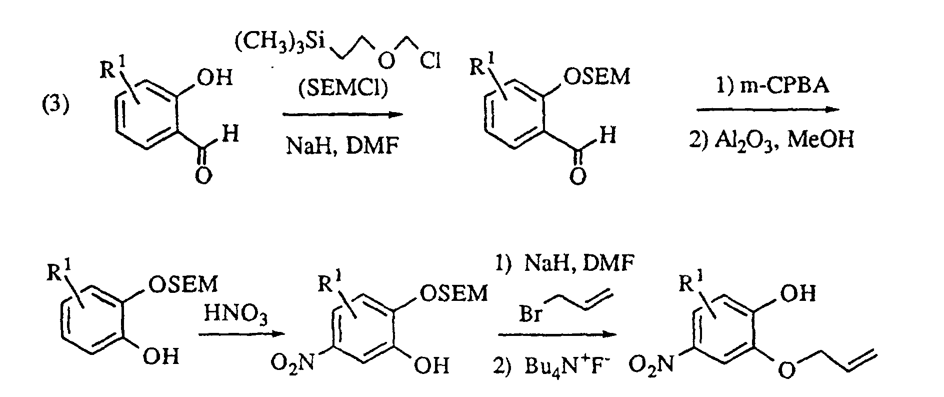

The processes described herein for the preparation of novel compounds of formula I form further aspects of the present invention.

The compounds of this invention are dopamine autoreceptor agonists; that is, they serve to modulate the synthesis and release of the neurotransmitter dopamine. These compounds are also partial agonists at the postsynaptic dopamine D₂ receptor, capable of functioning as either agonists or antagonists depending on the level of dopaminergic stimulation. They thus serve to modulate dopaminergic neurotransmission and are thereby useful for treatment of disorders of the dopaminergic system, such as schizophrenia, schizoaffective disorder, Parkinson's disease, Tourette's syndrome and hyperprolactinemia and in the treatment of drug addiction such as the addiction to ethanol or cocaine and related illnesses.

The antipsychotic activity of the compounds of the invention was established by a determination of functional antagonism of dopamine receptors *in vivo*, specifically the compounds' ability to reduce mouse locomotor activity according to the method of Martin and Bendensky, J. Pharmacol. Exp. Therap. 229: 706-711, 1984, in which mice (male, CF-1, Charles River, 20-30 g) were injected with vehicle or various doses of each drug and locomotor activity was measured for 30 minutes using automated infrared activity monitors (Omnitech - 8 x 8 inch open field) located in a darkened room. ED₅₀'s were calculated from the horizontal activity counts collected from 10 to 20 minutes after dosing using a nonlinear regression analysis with inverse prediction. When examined in this assay, the compounds of this invention produced ED₅₀'s of less than 5 mg/kg, sc.

Affinity for the dopamine D2 receptor was established by a modification of the standard experimental test procedure of Seemen and Schaus, European Journal of Pharmacology 203: 105-109, 1991, wherein homogenized rat striatal brain tissue is incubated with ³H-quinpirole and various concentrations of test compound, filtered and washed and counted in a Betaplate scintillation counter. The results of this testing with compounds representative of this invention are given below.

| Compound | D₂.Receptor Affinity (IC₅₀ (nM)) |
|---|---|
| Example 1 | 0.35 |
| Example 2 | 0.73 |
| Example 3 | 3.20 |
| Example 4 | 4.08 |
| Example 5 | 1.20 |
| Example 6 | 4.06 |
| Example 7 | 0.23 |

Hence, the compounds of this invention have potent affinity for dopamine receptors and produce a functional antagonism of dopamine receptors *in vivo* and thus are useful in the treatment of dopaminergic disorders such as schizophrenia, schizoaffective disorder, Parkinson's disease, Tourette's syndrome, hyperprolactinemia and drug addiction.

The compounds of this invention may be administered orally or parenterally, neat or in combination with conventional pharmaceutical carriers. Applicable solid carriers can include one or more substances which may also act as flavoring agents, lubricants, solubilizers, suspending agents, fillers, glidants, compression aids, binders or tablet-disintergrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be either liquid or solid composition form.

Preferably the pharmaceutical composition is in unit dosage form, e.g. as tablets or capsules. In such form, the composition is sub-divided in unit dose containing appropriate quantities of the active ingredient; the unit dosage forms can be packaged compositions, for example packeted powders, vials, ampoules, prefilled syringes or sachets containing liquids. The unit dosage form can be, for example, a capsule or tablet itself, or it can be the appropriate number of any such compositions in package form.

The dosage to be used in the treatment of a specific psychosis must be subjectively determined by the attending physician. The variables involved include the specific psychosis and the size, age and response pattern of the patient. Based upon the activity profile and potency of the compounds of this invention compared to the clinically useful antipsychotic risperidone, it is considered that a starting dose of about 10 mg per day with gradual in crease in the daily dose to about 200 mg per day will provide the desired dosage level in the human.

Thus, in a further aspect of the present invention there is provided a pharmaceutical composition comprising a compound of formula (I) as defined herein and a pharmaceutically acceptable carrier therefor. In a yet further aspect of the present invention there is provide a compoupnd of formula I for use as a pharmaceutical thereapeutic substance, particularly in respect of diseases of the dopaminergic system, in particular, schizophrenia or a schizoaffective disorders.

Furthermore, there is provided a method of treatment of diseases of brain dopamine dysregulation, for example, for treatment of schizophrenia or a schizoaffective disorder, which comprises administering, orally or parenterally, to a subject suffering from such a disorder of the dopaminergic system, an amount of a compound of formula I.

The following examples illustrate the production of representative compounds of this invention.

### INTERMEDIATE 1

### 3-Allyloxy-4-methoxynitrobenzene

97.5 g (0.51 mole) of the sodium salt of 5-nitroguaiacol was dissolved in one liter of DMF and 1.5 equivalents of allyl bromide added. The reaction was heated to 65°C for two hours, after which time much of the dark color had discharged and tlc (1:1 CH₂Cl₂/hexane) indicated loss of starting material. The solvent was concentrated in vacuum and the residue washed with water. The product was isolated by filtration and dried in a vacuum. This gave 112 g of pale yellow solid. A sample recrystallized from methanol, gave m.p. 93-94 °C.

### INTERMEDIATE 2

### 2-Allyloxy-4-nitrophenol

To one liter of dimethyl sulfoxide was added 750 ml of 2 N aqueous sodium hydroxide and the mixture was heated to 65°C. The pale yellow solid 3-allyloxy-4-methoxynitrobenzene prepared above was added in portions over a 30 minute period and then the temperature was raised to 95°C and maintained for 3 hours, after which time the starting material had been consumed. The mixture was allowed to cool and poured into a mixture of 1L ice and 1L 2 N HCl. 73 Grams of crude but homogeneous (by tlc 1:1 CH₂Cl₂/hexane) desired product was isolated as a light brown solid by filtration. This material was subsequently dissolved in 1:1 hexane/methylene chloride and filtered through silica gel to give 68 g of pale yellow solid, which, when recrystallized from ethyl/acetate/hexane, gave m.p. 61-62 °C. The aqueous mother liquors from the initial crystallization above were extracted with 2L of ethyl acetate. This was dried over sodium sulfate, filtered and evaporated to a dark oil. Column chromatography on silica with 1:1 CH₂Cl₂/hexane gave an additional 12 g of the title compound as a yellow solid. Elution with 2% MeOH in CHCl₃ gave 12 g of a dark oil which slowly crystallized in vacuum. This proved to be the Claisen product, 3-allyl-4-nitrocatechol.

### INTERMEDIATE 3

### 2-(2-Allyloxy-4-nitrophenoxymethyl)-oxirane

20 g (0.50 mole) of 60% NaH/mineral oil was placed in a two liter flask and washed with 500 ml of hexane. 1L of DMF was added, followed by 77 g (0.40 mole) of the 2-allyloxy-4-nitrophenol prepared in the previous step. Addition of the phenol was performed in portions under argon. After stirring the mixture for 30 minutes at room temperature under argon, 108 g (0.48 moles) of (R)-glycidyl tosylate was added and the mixture heated at 70-75°C under nitrogen overnight. Upon cooling, the DMF was removed in vacuum and replaced with one liter of methylene chloride. This was washed with 500 ml portions of 2 N HCl, saturated sodium bicarbonate and saturated brine and dried over sodium sulfate. The mixture was filtered, concentrated to an oil in vacuum and column chromatographed on silica gel using 1:1 hexane/methylene chloride as eluant. This gave 43 g of product contaminated with traces of the two starting materials, followed by 21 g of pure product as a pale yellow solid. The impure material was recrystallized from 1.2L of 10% ethyl acetate/hexane to give 34 g of pure (homogeneous on silica gel tlc with 1:1 hexane/methylene chloride) (R)-2-(2-allyloxy-4-nitrophenoxymethyl)-oxirane (m.p. 64 °C).

| | | | |
|---|---|---|---|
| Elemental Analysis for: C₁₂H₁₃NO₅ | | | |
| Calc'd | C, 57.37; | H, 5.21; | N, 5.58 |
| Found | C, 57.50; | H, 5.21; | N, 5.43 |

### INTERMEDIATE 4

### (8-Allyl-7-nitro-2,3-dihydro-benzo(1,4)dioxin-2-yl)-methanol

(R)-2-(2-Allyloxy-4-nitrophenoxymethyl)-oxirane (20 g, 80 mmoles) prepared as above was heated at 155°C in mesitylene for 24 hours under nitrogen. Filtration of the black solid which formed gave 1.5 g of very polar material. Evaporation of the solvent in vacuum followed by column chromatography on silica gel with methylene chloride as eluant gave 10 g of recovered starting material and 7.5 g of the desired rearranged (S)-(8-allyl-7-nitro-2,3-dihydro-benzo(1,4)dioxin-2-yl)-methanol, which slowly crystallized on standing in vacuum (m.p. 67 °C). The yield based on recovered starting material is 75%.

| | | | |
|---|---|---|---|
| Elemental Analysis for: C₁₂H₁₃NO₅ | | | |
| Calc'd | C, 57.37; | H, 5.21; | N, 5.58 |
| Found | C, 57.26; | H, 5.20; | N, 5.35 |

### INTERMEDIATE 5

### Toluene-4-sulfonic acid allyl-7-nitro-2,3-dihydro-benzo(1,4)dioxin-2-yl-methyl ester

9.55 g (38.0 mmole) of (S)-(8-allyl-7-nitro-2,3-dihydro-benzo(1,4)dioxin-2-yl)-methanol was dissolved in 465 ml of pyridine, 29.0 g (152 mmole) of p-toluenesulfonyl chloride was added and the mixture stirred at room temperature under nitrogen overnight. Water was then added to quench the excess tosyl chloride and the solvent was removed in vacuum and replaced with methylene chloride. This solution was washed with 2 N HCl, with saturated sodium bicarbonate, and with saturated brine, and dried over magnesium sulfate. Filtration, evaporation in vacuum and column chromatography on silica gel with 1:1 hexane/methylene chloride as eluant gave 12.6 g (92%) of toluene-4-sulfonic acid (R)-allyl-7-nitro-2,3-benzo(1,4)dioxin-2-ylmethyl ester, which slowly crystallized to a tan solid (m.p. 60-62 °C) upon standing.

| | | | |
|---|---|---|---|
| Elemental Analysis for: C₁₉H₁₉NO₇ S | | | |
| Calc'd | C, 56.29; | H, 4.72; | N, 3.45 |
| Found | C, 56.13; | H, 4.58; | N, 3.44 |

### INTERMEDIATE 6

### (6-Nitro-3-(toluene-4-sulfonyloxymethyl)-2,3-dihydro-benzo(1,4)dioxin-5-yl)-acetic acid

Potassium permanganate (11.7 g, 0.074 mole) was placed in a flask which was equipped with a mechanical stirrer, a dropping funnel, and an ice bath. To this was added 150 ml of H₂0 and tetrabutylammonium chloride (1.0 g, 3.7 mmole) with stirring. The toluene-4-sulfonic acid (R)-allyl-7-nitro-2,3-benzo(1,4)dioxin-2-ylmethyl ester prepared above dissolved in 100 ml of benzene was slowly added through a dropping funnel and the reaction mixture was stirred further for 30 minutes in an ice bath. The ice bath was then removed and the mixture was stirred for 24 hours at room temperature. 30 g of sodium bisulfite was added to the mixture with good stirring in an ice bath and acidified with concentrated HCl until pH < 3. The acidified clear yellow solution was then extracted with ethyl acetate and the combined extracts were dried over anhydrous magnesium sulfate. The concentrated residue was chromatographed on a silica gel column using ethyl acetate as an eluant to give 6.3 g (60%) of (R)-(6-nitro-3-(toluene-4-sulfonyloxymethyl)-2,3-dihydro-benzo(1,4)-dioxin-5-yl)-acetic acid_as a pale yellow solid. Crystallization from methylene chloride gave a light yellow solid with m.p. 158-159 °C.

| | | | |
|---|---|---|---|
| Elemental Analysis for: C₁₈H₁₇NO₉S • 1/4 H₂O | | | |
| Calc'd | C, 50.52; | H, 4.12; | N, 3.27 |
| Found | C, 50.51; | H, 3.83; | N, 3.12 |

### INTERMEDIATE 7

### 2-(Toluene-4-sulfonyloxymethyl)-2,3,8,9-tetrahydro-7H-1,4-dioxino[2,3-e]-indol-8-one

The carboxylic acid ( 6.0 g, 0.0142 mole) obtained above was ground into a fine powder. To this was added 300 ml of water and 5 ml of 2.5 N NaOH until the pH was 8, and the heterogeneous solution was stirred for 30 minutes until the solid was evenly dispersed. 1.0 g of 10% Pd on carbon was then added and the mixture was hydrogenated on a Parr shaker for 24 hours at 52 psi of hydrogen. The catalyst was filtered off and washed with water. The volume of the filtrate was then reduced by half and acidified with 15 ml of concentrated HCl while stirring in an ice bath to precipitate a white solid acid product, (R)-(6-amino-3-(toluene-4-sulfonyloxymethyl)-2,3-dihydro-benzo(1,4)dioxin-5-yl)-acetic acid. This heterogeneous solution was then heated at 50° C for 24 hours. As time passed, tlc (5% methanol/CH₂Cl₂ on silica gel) showed that the amino acid was slowly replaced with lactam, and the reaction mixture became clear briefly and then the title compound started to precipitate as a white solid. After the mixture was cooled to room temperature and stirred for an additional hour, the white solid was filtered, washed with diethyl ether and dried in a vacuum at room temperature. The product (R)-2-(toluene-4-sulfonyloxymethyl)-2,3,8,9-tetrahydro-7H-1,4-dioxino[2,3-e]indol-8-one_(m.p. 225-227 °C) was pure without further recrystallization and weighed 4.2 g (79%).

| | | | |
|---|---|---|---|
| Elemental Analysis for: C₁₈H₁₇NO₆ S | | | |
| Calc'd | C, 57.59; | H, 4.57; | N, 3.73 |
| Found | C, 57.34; | H, 4.55; | N, 3.69 |

### EXAMPLE 1

### 2-(3,4-Dihydro-1H-isoquinolin-2-ylmethyl)-2,3,8,9-tetrahydro-7H-1,4-dioxino[2,3-e]indol-8-one

(R)-2-(Toluene-4-sulfonyloxymethyl)-2,3,8,9-tetrahydro-7H-1,4-dioxino[2,3-e]indol-8-one (1.05 g, 2.80 mmole) and tetrahydroisoquinoline (1.60 ml, 12.6 mmole) were combined in 30 ml of dry DMSO and heated to 85 °C for 4.5 hours under a nitrogen atmosphere. The reaction was cooled and taken into 400 ml of 1:1 hexane/ethyl acetate. This was washed with 200 ml of water, with 200 ml of saturated brine, dried over MgSO₄, filtered and concentrated to yield an oil. This oil was column chromatographed on silica gel using 0.75% methanol/CH₂Cl₂ as eluant to give the free base of the title compound as a yellow oil (0.77 g, 82%). This oil was crystallized from ethanol with the addition of a solution of fumaric acid in hot ethanol to give 0.61 g of the (S) enantiomer of the title compound as a light yellow solid fumarate, m.p. 195-196 °C.

| | | | |
|---|---|---|---|
| Elemental Analysis for: C₂₀H₂₀N₂ S • C₄H₄O₄ | | | |
| Calc'd | C, 63.71; | H, 5.35; | N, 6.19 |
| Found | C, 63.39; | H, 5.39; | N, 6.01 |

### EXAMPLE 2

### 2-[4-(4-Fluoro-benzoyl)-piperidin-1-ylmethyl]-2,3,8,9-tetrahydro-7H-1,4-dioxino[2,3-e]indol-8-one

(R)-2-(Toluene-4-sulfonyloxymethyl)-2,3,8,9-tetrahydro-7H-1,4-dioxino[2,3-e]indol-8-one (1.03 g, 2.75 mmole), 4-(4-fluorobenzoyl)piperidine p-toluenesulfonate (4.68 g, 12.4 mmole) and diisopropylethylamine (2.15 ml, 12.3 mmole) were combined in 70 ml of dry DMSO and heated to 85 °C for 5 hours under a nitrogen atmosphere. The reaction was cooled and taken into 400 ml of 1:1 hexane/ethyl acetate. This was washed with 200 ml of water, with 200 ml of saturated brine, dried over MgSO₄, filtered and concentrated to yield an oil. This oil was column chromatographed on silica gel using 0.75% methanol/CH₂Cl₂ as eluant to give the free base of the title compound as a yellow oil (0.40 g, 40%). This oil was crystallized from ethanol with the addition of a solution of fumaric acid in hot ethanol to give 0.37 g of the (S) enantiomer of the title compound as a light yellow solid fumarate, m.p. 237-238 °C.

| | | | |
|---|---|---|---|
| Elemental Analysis for: C₂₃H₂₃FN₂O₄ S • C₄ H₄O₄ | | | |
| Calc'd | C, 61.59; | H, 5.17; | N, 5.32 |
| Found | C, 61.41; | H, 4.95; | N, 5.30 |

### EXAMPLE 3

### 2-(4-Oxo-1-phenyl-1,3,8-triaza-spiro[4,5]dec-8-ylmethyl)-2,3,8,9-tetrahydro-7H-1,4-dioxino-[2,3-e]indol-8-one

(R)-2-(Toluene-4-sulfonyloxymethyl)-2,3,8,9-tetrahydro-7H-1,4-dioxino[2,3-e]indol-8-one (1.04 g, 2.77 mmole) and 1-phenyl-1,3,8-triazaspirodecan-4-one (2.89 g, 12.5 mmole) were combined in 40 ml of dry DMSO and heated to 85 °C for 4 hours under a nitrogen atmosphere. The reaction was cooled and taken into 400 ml of 1:1 hexane/ethyl acetate. This was washed with 200 ml of water, with 200 ml of saturated brine, dried over MgSO₄, filtered and concentrated to yield an oil. This oil was column chromatographed on silica gel using 2.5% methanol/CH₂Cl₂ as eluant to give the free base of the title compound as a yellow oil (0.40 g, 33%). This oil was crystallized from ethanol with the addition of a solution of fumaric acid in hot ethanol to give 0.31 g of the (S) enantiomer of the title compound as a light yellow solid hemifumarate, hemihydrate, m.p. 264-265.5 °C.

| | | | |
|---|---|---|---|
| Elemental Analysis for: C₂₄H₂₆N₄ O₄ • 0.5 C₄ H₄ O₄ • 0.5 H₂O | | | |
| Calc'd | C, 62.26; | H, 5.83; | N, 11.17 |
| Found | C, 62.38; | H, 5.75; | N, 11.01 |

### EXAMPLE 4.

### 2-[4-(2-Oxo-2,3-dihydro-benzimidazol-1-yl)-piperidin-1-ylmethyl}-2,3,8,9-tetrahydro-7H-1,4-dioxino[2,3-e]indol-8-one

(R)-2-(Toluene-4-sulfonyloxymethyl)-2,3,8,9-tetrahydro-7H-1,4-dioxino[2,3-e]indol-8-one (1.04 g, 2.77 mmole) and 4-(2-oxo-2,3-dihydro-benzimidazol-1-yl)-piperidine (2.71 g, 12.5 mmole) were combined in 40 ml of dry DMSO and heated to 85 °C for 4 hours under a nitrogen atmosphere. The reaction was cooled and taken into 400 ml of 1:1 hexane/ethyl acetate. This was washed with 200 ml of water, with 200 ml of saturated brine, dried over MgSO₄, filtered and concentrated to yield an oil. This oil was column chromatographed on silica gel using 1% methanol/CH₂Cl₂ as eluant to give the free base of the title compound as a yellow oil (0.65 g, 53%). This oil was crystallized from ethanol with the addition of a solution of fumaric acid in hot ethanol to give 0.61 g of the (S) enantiomer of the title compound as a light yellow solid fumarate, hemihydrate, m.p. 262-263.5 °C.

| | | | |
|---|---|---|---|
| Elemental Analysis for: C₂₃H₂₄N₄O₄ • C₄H₄O₄ • 0.5 H₂O | | | |
| Calc'd | C, 59.44; | H, 5.36; | N, 10.27 |
| Found | C, 56.11; | H, 5.31; | N, 10.24 |

### EXAMPLE 5

### 2-(4-Phenyl-3,6-dihydro-2H-pyridin-1-ylmethyl)-2,3,8,9-tetrahydro-7H-1,4-dioxino[2,3-e]indol-8-one

(R)-2-(Toluene-4-sulfonyloxymethyl)-2,3,8,9-tetrahydro-7H-1,4-dioxino[2,3-e]indol-8-one (1.04 g, 2.77 mmole), 4-phenyltetrahydropyridine hydrochloride (2.45 g, 12.5 mmole) and diisopropylethylamine (2.20 ml, 12.5 mmole) were combined in 50 ml of dry DMSO and heated to 85 °C for 5 hours under a nitrogen atmosphere. The reaction was cooled and taken into 400 ml of 1:1 hexane/ethyl acetate. This was washed with 200 ml of water, with 200 ml of saturated brine, dried over MgSO₄, filtered and concentrated to yield an oil. This oil was column chromatographed on silica gel using 1.5% methanol/CH₂Cl₂ as eluant to give 0.15 g of the (S) enantiomer of the free base of the title compound as a light yellow solid one-quarter hydrate, m.p. 264-265 °C.

| | | | |
|---|---|---|---|
| Elemental Analysis for: C₂₂H₂₂N₂O₃ • 0.25 H₂O | | | |
| Calc'd | C, 72.01; | H, 6.18; | N, 7.63 |
| Found | C, 72.28; | H, 6.08; | N, 7.65 |

### EXAMPLE 6

### 2-[4-(1H-Indol-4-yl)-piperazin-1-ylmethyl]-2,3,8,9-tetrahydro-7H-1,4-dioxino[2,3-e]indol-8-one

(R)-2-(Toluene-4-sulfonyloxymethyl)-2,3,8,9-tetrahydro-7H-1,4-dioxino[2,3-e]indol-8-one (1.0 g, 2.7 mmole) and 4-(1H-indol-4-yl)-piperazine (2.0 g, 10 mmole) were combined in 30 ml of dry DMSO and heated to 80 °C for 4 hours under an argon atmosphere. After cooling to room temperature, the mixture was diluted with 400 ml of 1:1 ethyl acetate/hexane and washed with 400 ml of saturated sodium bicarbonate solution, with two 250 ml portions of water and with saturated brine. The mixture was dried over sodium sulfate, filtered and concentrated in vacuum to yield an oil, which was column chromatographed on silica gel using 0.5% methanol/CHCl₃ as eluant. The free base of the title compound (0.80 g) thus obtained was crystallized from methanol with the addition of one equivalent of fumaric acid to give 077 g of the (S) enantiomer of the title compound as a white solid fumarate, m.p. 237-238 °C.

| | | | |
|---|---|---|---|
| Elemental Analysis for: C₂₃H₂₄N₄O₃ • C₄H₄O₄ | | | |
| Calc'd | C, 62.30; | H, 5.42; | N, 10.76 |
| Found | C, 62.02; | H, 5.38; | N, 10.70 |

### INTERMEDIATE 8

### (R)-2-Toluene-4-sulfonyloxymethyl)-6-fluoro-2,3-dihydrobenzo[1,4]dioxin

(S)-(6-flouro-2,3-dihydrobenzo(1,4 dioxin-2-yl)-methanol (17 g, 92 mmole) was dissolved in one liter of pyridine. To this solution was added 38 g (0.20 mole) of p-toluenesulfonyl chloride and the mixture stirred at room temperature under nitrogen for three days. The reaction was cooled in an ice-water bath and to it was added slowly 10 ml of water. The mixture was stirred at room temperature for 2 hours and then the solvent was removed under vacuum and replaced with 800 ml of methylene chloride. This solution was washed twice with 500 ml of 1 N HCl (aq), with saturated aqueous sodium bicarbonate, and with saturated brine and dried over sodium sulfate. Filtration, evaporation in vacuum and column chromatography on silica gel with 50% hexane in dichloromethane as eluent gave 25.1 g (89%) of the title compound as an off-white solid. ¹H (CDCl₃) doublet, 7.86 δ (2 H); doublet, 7.32 δ (2 H); doublet of doublets, 6.65 δ (1 H); multiplet, 6.58 δ, (2 H); multiplet, 4.34 δ (1 H); multiplet, 4.20 δ (3 H); multiplet, 4.00 δ (1 H); singlet, 2.43 δ (3 H).

### INTERMEDIATE 9

### (R)-2-Toluene-4-sulfonyloxymethyl)-6-fluoro-7-pitro-2,3-dihydrobenzo[1,4]dioxin

(R)-2-Toluene-4-sulfonyloxymethyl)-6-fluoro-2,3-dihydrobenzo[1,4]dioxin (25.1 g, 74 mmole) was dissolved in 250 ml of dichloroethane and cooled to 0 °C in an ice/water bath. To this cooled solution was added dropwise over a 15 minute period a solution of nitric acid (sp. gr. 1.49) in 60 ml of dichloroethane. The mixture was stirred at 0 °C under nitrogen for two hours, after which time the reaction was quenched by the addition of 500 g of ice. The mixture was diluted to 700 ml with methylene chloride and washed with saturated aqueous sodium bicarbonate solution, with water, with saturated brine and dried over sodium sulfate. Filtration and evaporation in vacuum gave 25 g of crude product. This was column chromatographed on silica gel using 1:1 hexane/ethyl acetate as eluant to give 21 g of the title compound as a yellow solid. ¹H (CDCl₃) doublet, 7.80 δ (2 H); doublet, 7.50 δ (1 H); doublet, 7.38 δ (2 H); doublet, 6.76 δ (1 H); multiplet, 4.40 δ (2 H); multiplet, 4.25 δ (2 H); multiplet, 4.15 δ (1 H); singlet, 2.43 δ (3 H).

### INTERMEDIATE 10

### (R)-2-Toluene-4-sulfonyloxymethyl)-6-fluoro-7-amino-2,3-dihydrobenzo[1,4]dioxin

(R)-2-Toluene-4-sulfonyloxymethyl)-6-fluoro-7-nitro-2,3-dihydrobenzo[1,4]-dioxin (21 g, 55 mmole) was added to a suspension of 2.0 g of 10% palladium on carbon in 250 ml of methanol. To this was added 15 ml of 4 N isopropanolic HCl. The mixture was hydrogenated for 20 hours using a Parr apparatus at 50-60 psi of hydrogen. The mixture was then filtered through celite and the catalyst washed with additional methanol. The filtrate was concentrated in vacuum to give 21.4 g of the title compound as a gray solid hydrochloride. ¹H (DMSO-d₆) doublet, 7.80 δ (2 H); doublet, 7.47 δ (2 H); doublet, 6.95 δ (1 H); doublet, 6.85 δ (1 H); multiplet, 4.40 δ (1 H); multiplet, 4.25 δ (3 H); multiplet, 4.00 δ (1 H); singlet, 2.40 δ (3 H).

### INTERMEDIATE 11

### (R)-2-(Toluene-4-sulfonyloxymethyl)-6-fluoro-2,3,8,9-tetrahydro-7H-1,4-dioxino[2,3-e]indol-8-one

In a three-neck flask equipped with a dropping funnel, thermometer and a nitrogen inlet was placed 6.15 ml (48.0 mmole) of ethyl methylthioacetate and 65 ml of dry methylene chloride. The solution was cooled to -78 °C by means of a dry ice/isopropanol bath and to it was added dropwise over a 5 minute period a solution of 3.80 g (47.0 mmole) of sulfuryl chloride in 30 ml of methylene chloride. The reaction was maintained at -78 °C for 30 minutes. To the mixture was added dropwise over a one hour period a solution of (R)-2-toluene-4-sulfonyloxymethyl)-6-fluoro-7-amino-2,3-dihydrobenzo[1,4]dioxin (15.7 g, 45.0 mmole) and Proton Sponge (11.7 g, 47.0 mmole) in 150 ml of methylene chloride. The mixture was stirred a -78 °C for two hours, then 9.5 g (54 mmole) of diisopropylethylamine in 20 ml of dichloromethane added dropwise over 10 minutes and stirring continued for an additional hour at -78 °C, after which the reaction was allowed to come to room temperature and stirred for 8 hours under nitrogen. The resulting solution was diluted to 500 ml with methylene chloride and washed with saturated brine, dried over magnesium sulfate, filtered and concentrated in vacuum to a brown oil. This was dissolved in 200 ml of glacial acetic acid and stirred for 10 hours at room temperature under a nitrogen atmosphere. The solvent was then removed in vacuum and replaced with 500 ml of methylene chloride. The mixture was washed with 300 ml of saturated aqueous sodium bicarbonate and 300 ml saturated brine, dried over magnesium sulfate, filtered and concentrated in vacuum to a brown oil, which was column chromatographed on silica gel using 2% methanol in methylene chloride as eluant. The light brown solid (13.0 g, 66%) thus obtained was dissolved in 200 ml of tetrahydrofuran (THF) and added to a suspension in 600 ml of THF of approximately 200 g of Raney nickel (Ra-Ni weighed as a slurry in water), which had been washed with water, with 0.5% aqueous acetic acid, again with water and finally with THF. The reaction was stirred at room temperature for 8 hours, then the solution decanted and the catalyst washed thoroughly with THF. The combined organic fractions were concentrated in vacuum and the product column chromatographed on silica gel using methylene chloride as eluant. The title compound (4.54 g, 40%) was isolated as an off-white solid, m.p. 205-206 °C.

| | | | |
|---|---|---|---|
| Elemental Analysis for: C₁₈H₁₆FNO₆S • 0.25 H₂O | | | |
| Calc'd | C, 54.34; | H, 4.18; | N, 3.52 |
| Found | C, 54.12; | H, 4.24; | N, 3.41 |

### EXAMPLE 7

### 2-(3,4-Dihydro-1H-isoquinolin-2-ylmethyl)-6-fluoro-2,3,8,9-tetrahydro-7H-1,4-dioxino[2,3-e]indol-8-one

(R)-2-(Toluene-4-sulfonyloxymethyl)-6-fluoro-2,3,8,9-tetrahydro-7H-1,4-dioxino[2,3-e]indol-8-one (1.0 g, 2.5 mmole) and tetrahydroisoquinoline (1.3 g, 10 mmole) were combined in 30 ml of dry DMSO and heated at 80-90 °C for 4 hours under an argon atmosphere. After cooling to room temperature, the mixture was diluted with 500 ml of 1:1 ethyl acetate/hexane and washed with 250 ml of saturated aqueous sodium bicarbonate and with two 250 ml portions of water, dried over sodium sulfate, filtered and concentrated in vacuum. The residue was column chromatographed on silica gel using 0.5% methanol/CHCl₃ as eluant to give the free base of the title compound as a pale yellow oil. This was crystallized from ethanol with the addition of one equivalent of fumaric acid to give 0.79 g of the (S) enantiomer of the title compound as a pale orange solid fumarate, m.p. 219--220 °C.

| | | | |
|---|---|---|---|
| Elemental Analysis for: C₂₀H₁₉FN₂O₃ • C₄H₄O₄ | | | |
| Calc'd | C, 61.27; | H, 4.93; | N, 5.95 |
| Found | C, 61.12; | H, 4.84; | N, 5.83 |

### EXAMPLE 8

### 2-[4-(1H-Indol-3-yl)-1-piperidinylmethyl]-6-fluoro-2,3,8,9-tetrahydro-7H-1,4-dioxino[2,3-e]indol-8-one

(R)-2-(Toluene-4-sulfonyloxymethyl)-6-fluoro-2,3,8,9-tetrahydro-7H-1,4-dioxino[2,3-e]indol-8-one (1.0 g, 2.5 mmole) and 4-(1H-indol-3-yl)piperidine (2.0 g, 10 mmole) are combined in 30 ml of dry DMSO and heated at 80-90 °C for 4 hours under an argon atmosphere. After cooling to room temperature, the mixture is diluted with 500 ml of 1:1 ethyl acetate/hexane and washed with 250 ml of saturated aqueous sodium bicarbonate and with two 250 ml portions of water, dried over sodium sulfate, filtered and concentrated in vacuum. The residue is column chromatographed on silica gel using 0.5% methanol/CHCl₃ as eluant to give the free base of the title compound. The product is crystallized from ethanol with the addition of one equivalent of fumaric acid to give the (S) enantiomer of the title compound as a fumarate salt.

### EXAMPLE 9

### 2-[4-(1,2-Benzisothiazol-3-yl)-1-piperazinylmethyl)-2,3,8,9-tetrahydro-7H-1,4-dioxino[2,3-e]indol-8-one

(R)-2-(Toluene-4-sulfonyloxymethyl)-2,3,8,9-tetrahydro-7H-1,4-dioxino[2,3-e]indol-8-one (1.0 g, 2.7 mmole) and 1-(1,2-benzisothiazol-3-yl)piperazine (2.2 g, 10 mmole) are combined in 30 ml of dry DMSO and heated at 80-90 °C for 4 hours under an argon atmosphere. After cooling to room temperature, the mixture is diluted with 500 ml of 1:1 ethyl acetate/hexane and washed with 250 ml of saturated aqueous sodium bicarbonate and with two 250 ml portions of water, dried over sodium sulfate, filtered and concentrated in vacuum. The residue is column chromatographed on silica gel using 0.5% methanol/CHCl₃ as eluant to give the free base of the title compound. The product is crystallized from ethanol with the addition of one equivalent of fumaric acid to give the (S) enantiomer of the title compound as a fumarate salt.

### EXAMPLE 10

### 2-[4-(6-Fluoro-1,2-benzisoxazol-3-yl)-1-piperidinylmethyl]-2,3,8,9-tetrahydro-7H-1,4-dioxino[2,3-e]indol-8-one

(R)-2-(Toluene-4-sulfonyloxymethyl)-2,3,8,9-tetrahydro-7H-1,4-dioxino-[2,3-e]indol-8-one (1.0 g, 2.7 mmole) and 4-(6-fluoro-1,2-benzisoxazol-3-yl)-1-piperidine (2.2 g, 10 mmole) are combined in 30 ml of dry DMSO and heated at 80-90 °C for 4 hours under an argon atmosphere. After cooling to room temperature, the mixture is diluted with 500 ml of 1:1 ethyl acetate/hexane and washed with 250 ml of saturated aqueous sodium bicarbonate and with two 250 ml portions of water, dried over sodium sulfate, filtered and concentrated in vacuum. The residue is column chromatographed on silica gel using 0.5% methanol/CHCl₃ as eluant to give the free base of the title compound. The product is crystallized from ethanol with the addition of one equivalent of fumaric acid to give the (S) enantiomer of the title compound as a fumarate salt.

### EXAMPLE 11

### 4-Fluoro-8-[4-(1H-indol-3-yl)-3,6-dihydro-2H-pyridin-1-ylmethyl]-1,3,7,8-tetrahydro-6,9-dioxa-3-aza-cyclopenta[a]paphthalen-2-one

(R)-2-(Toluene-4-sulfonyloxymethyl)-6-fluoro-2,3,8,9-tetrahydro-7H-1,4-dioxino[2,3-e]indol-8-one (0.92 g, 2.34 mmole) 4-(lH-indol-3-yl)-3,6-dihydro-2H-pyridine (1.30 g, 6.6 mmole) were combined in 40 ml of dry DMSO and heated at 80 °C for 5 hours under a nitrogen atmosphere. After cooling to room temperature, the mixture was diluted with 150 ml of water and extracted with 0.4% methanol in ethyl acetate. The organic extract was washed with 100 ml of saturated aqueous sodium bicarbonate solution, dried over magnesium sulfate, filtered and concentrated to a red oil in vacuum. The residue was column chromatographed on silica gel using first 10% dichloromethane/hexane, then dichloromethane and finally 2% methanol in dichloromethane to give the desired product as an oil contaminated with DMSO. The oil was redissolved in ethyl acetate and washed three times with 150 ml portions of water, dried over magnesium sulfate, filtered and concentrated to a yellow oil in vacuum. Addition of ethanol to the oil gave 0.25 g of the (S) enantiomer of the title compound as a yellow solid, m.p. 230 °C.

| | | | |
|---|---|---|---|
| Elemental Analysis for: C₂₄H₂₂FN₃O₃ • 0.5 H₂O | | | |
| Calc'd | C, 67.28; | H, 5.41; | N, 9.81 |
| Found | C, 67.18; | H, 5.34; | N, 9.86 |

## Claims

1. A compound of formula I 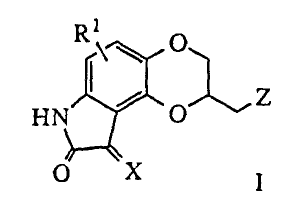 wherein
X is H₂ or O;
R¹ is hydrogen, hydroxy, halo, trifluoromethyl, trifluoromethoxy, alkyl of 1 to 6 carbon atoms, alkoxy of 1 to 6 carbon atoms, aralkoxy of 7 to 12 carbon atoms, alkanoyloxy of 2 to 6 carbon atoms, amino, mono- or di-alkylamino in which each alkyl group has 1 to 6 carbon atoms, alkanamido of 2 to 6 carbon atoms or alkanesulfonamido of 1 to 6 carbon atoms;
Z is defined by 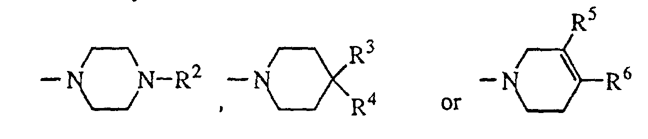 wherein
R² is hydrogen, alkyl of 1 to 6 carbon atoms, cycloalkyl of 3 to 8 carbon atoms, phenyl optionally substituted with R¹ as defined above; ω-phenylalkyl or ω-diphenylalkyl, in which the alkyl chain contains 1 to 4 carbon atoms and the phenyl ring is optionally substituted with R¹ as defined above, or R² is naphthyl, indolyl, indazolyl, thienyl, pyridinyl, pyrimidinyl, quinolinyl, benzoisothiazolyl or benzisoxazolyl, each optionally substituted with R¹ as defined above;
R³ is hydrogen and R⁴ is hydrogen, 1-benzimidazolyl-2-one, indolyl, benzoisothiazolyl or benzisoxazole, each optionally substituted with R¹ as defined above, or R⁴ is -Y-Ar, in which Y is C=O, CHOH, or (CH₂)ₘ, wherein m is 0 to 4, and Ar is phenyl, optionally substituted with R¹ as defined above,
or R³ and R⁴, taken together with the carbon atom to which they are attache form 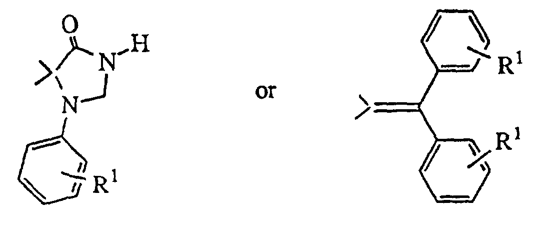
R⁵ is hydrogen and R⁶ is phenyl, naphthyl, thienyl, indolyl, benzoisothiazolyl or benzisoxazolyl, each optionally substituted with R¹ as defined above,
or R⁵ and R⁶, taken together with the carbon atoms to which they are attached complete a benzene ring optionally substituted with R¹;
or a pharmaceutically acceptable salt thereof.

2. A compound of Claim 1 in which R² is phenyl, naphthyl, indolyl, indazolyl, thienyl, pyridinyl, pyrimidinyl, quinolinyl, benzoisothiazolyl or benzisoxazolyl, each optionally substituted with R¹ as defined in Claim 1, R³ is hydrogen and R⁴ is 1-benzimidazolyl-2-one, indolyl, benzoisothiazolyl or benzisoxazolyl, each optionally substituted with R¹ as defined in Claim 1, or R⁴ is -Y-Ar, in which Y is C=O, and Ar is phenyl, optionally substituted with R¹ as defined in Claim 1, R⁵ is hydrogen and R⁶ is phenyl, indolyl, benzoisothiazolyl or benzisoxazolyl, each optionally substituted with R¹ as defined in claim 1, or R⁵ and R⁶, taken together with the carbon atoms to which they are attached complete a benzene ring optionally substituted with R¹; or a pharmaceutically acceptable salt thereof.

3. A compound of Claim 1 in which X is H₂, R² is phenyl, indolyl, pyridinyl, pyrimidinyl, quinolinyl, benzoisothiazolyl or benzisoxazolyl, each optionally substituted with R¹ as defined in claim 1, R⁵ is hydrogen and R⁶ is phenyl, optionally substituted with R¹ as defined above, or R⁵ and R⁶, taken together with the carbon atoms to which they are attached complete a benzene ring optionally substituted with R¹; or a pharmaceutically acceptable salt thereof.

4. The compound of Claim 1 which is 2-(3,4-dihydro-1H-isoquinolin-2-ylmethyl)-2,3,8,9-tetrahydro-7H-1,4-dioxino[2,3-e]indol-8-one or a pharmaceutically acceptable salt thereof.

5. The compound of Claim 1 which is 2-[4-(4-fluoro-benzoyl)-piperidin-1-ylmethyl]-2,3,8,9-tetrahydro-7H-1,4-dioxino[2,3-e]indol-8-one or a pharmaceutically acceptable salt thereof.

6. The compound of Claim 1 which is 2-(4-oxo-1-phenyl-1,3,8-triazaspiro[4,5]dec-8-ylmethyl)-2,3,8,9-tetrahydro-7H-1,4-dioxino-[2,3-e]indol-8-one or a pharmaceutically acceptable salt thereof.

7. The compound of Claim 1 which is 2-[4-(2-oxo-2,3-dihydro-benzimidazol-1-yl)-piperidin-1-ylmethyl}-2,3,8,9-tetrahydro-7H-1,4-dioxino[2,3-e]indol-8-one or a pharmaceutically acceptable salt thereof.

8. The compound of Claim 1 which is 2-(4-phenyl-3,6-dihydro-2H-pyridin-1-ylmethyl)-2,3,8,9-tetrahydro-7H-1,4-dioxino[2,3-e]indol-8-one or a pharmaceutically acceptable salt thereof.

9. The compound of Claim 1 which is 2-[4-(1H-indol-4-yl)-piperazin-1-ylmethyl]-2,3,8,9-tetrahydro-7H-1,4-dioxino[2,3-e]indol-8-one or a pharmaceutically acceptable salt thereof.

10. The compound of Claim 1 which is 2-(3,4-dihydro-1H-isoquinolin-2-ylmethyl)-6-fluoro-2,3,8,9-tetrahydro-7H-1,4-dioxino[2,3-e]indol-8-one or a pharmaceutically acceptable salt thereof.

11. The compound of Claim 1 which is 2-[4-(lH-indol-3-yl)-1-piperidinylmethyl]-6-fluoro-2,3,8,9-tetrahydro-7H-1,4-dioxino[2,3-e]indol-8-one or a pharmaceutically acceptable salt thereof.

12. The compound of Claim 1 which is 2-[4-(1,2-benzisothiazol-3-yl)-1-piperazinylmethyl)-2,3,8,9-tetrahydro-7H-1,4-dioxino[2,3-e]indol-8-one or a pharmaceutically acceptable salt thereof.

13. The compound of Claim 1 which is 2-[4-(6-fluoro-1,2-benzisoxazol-3-yl)-1-piperidinylmethyl]-2,3,8,9-terrahydro-7H-1,4-dioxino[2,3-e]indol-8-one or a pharmaceutically acceptable salt thereof.

14. The compound of Claim 1 which is 4-fluoro-8-[4-(1H-indol-3-yl)-3,6-dihydro-2H-pyridin-1-ylmethyl]-1,3,7,8-tetrahydro-6,9-dioxa-3-aza-cyclopenta[a]naphthalen-2-one or a pharmaceutically acceptable salt thereof.

15. Use of a compound of formula I as defined in any of claims 1 to 14 for the preparation of a medicament for the treatment of diseases of brain dopamine dysregulation which treatment comprises administering the medicament, orally or parenterally, to a subject suffering from such a disorder of the dopaminergic system.

16. Use of a compound according to Claim 15, for treatment of schizophrenia or a schizoaffective disorder, which comprises administering, orally or parenterally, to a subject suffering from dysregulation of the dopaminergic system, an amount of a compound of formula I.

17. A pharmaceutical composition comprising a compound of formula (I) as defined in any of claims 1 to 14 and a pharmaceutically acceptable carrier therefor.

18. A compound as defined in any of claims 1 to 14 for use as a pharmaceutical or therapeutic substance.

19. A compound as defined in any of claims 1 to 14 for use in the treatment of diseases of brain dopamine dysregulation.

20. A compound according to claim 19 for use in the treatment of schizophrenia or a schizoaffective disorder.

21. Process for the preparation of compounds of formula I as defined in any of claims 1 to 14 comprising :
a. alkylating an appropriately substituted nitroguaiacol with an allyl halide;
b. demethylating the resulting compound to form the compound of formula : 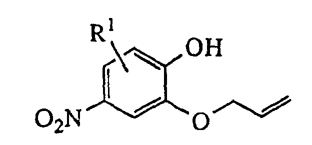
c. alkylating with glycidyl tosylate or an epihalohydrin in the presence of a base to form the compound of formula: 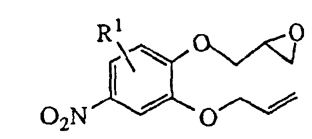
d. heating the resultant compound in a high boiling solvent to form the compound of formula: 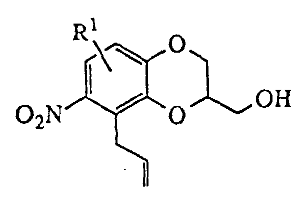
e. converting to a tosylate by reaction with p-toluenesulfonyl chloride in the presence of pyridine or alternately to a halide by reaction with carbon tetrabromide or carbon tetrachloride in combination with triphenylphosphine;
f. converting the allyl side chain to an acetic acid moiety by oxidative cleavage;
g. reducing the nitro group to an amine;
h. cyclicising the amine and the acid group to lactam using a dilute acid;
i. replacing the tosylate or halide with the appropriate azaheterocycle in the presence of a high boiling solvent to form the compound of formula (I); and optionally forming a pharmaceutically acceptable salt thereof.

22. Process for the preparation of compounds of formula I as defined in any of claims 1 to 14 comprising :
a. alkylating an appropriately substituted salicylaldehyde with an allyl halide to form the compound of formula ; 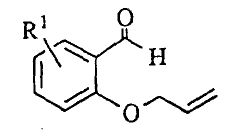
b. converting the aldehyde to a phenol by treatment with a peroxybenzoic acid followed by cleavage of the intermediate ester : 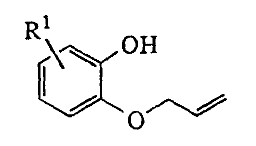
c. alkylating with glycidyl tosylate or an epihalohydrin in the presence of a base to form the compound of formula: 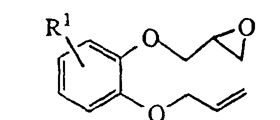
d. heating in a high boiling solvent followed by cyclicisation to form the compound of formula : 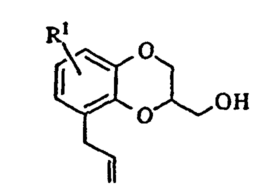
e. converting to a tosylate by reaction with p-toluenesulfonyl chloride in the presence of pyridine or alternately to a halide by reaction with carbon tetrabromide or carbon tetrachloride in combination with triphenylphosphine;
f. converting the allyl side chain to an acetic acid group by oxidative cleavage;
g. introducing a nitro group by treatment with nitric acid in a solvent;
h. reducing the nitro group to an amine;
i. cyclising the amine and the acid group to the corresponding lactam;
j. replacing the tosylate or halide with the appropriate azaheterocycle in the presence of a high boiling solvent to form the compound of formula I; and optionally forming a pharmaceudcally acceptable salt thereof.

23. Process for the preparation of compounds of formula I as defined in any of claims 1 to 14 comprising :
a. converting an appropriately substituted benzodioxan methanol of formula : 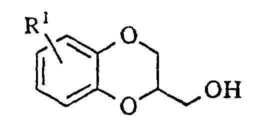 to a methyl tosylate by reaction with p-toluenesulfonyl chloride in the presence of pyridine;
b. reacting the resulting compound with nitric acid in a solvent to obtain the corresponding nitro substituted compound of formula : 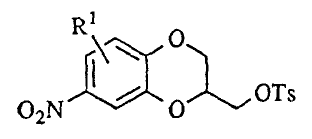
c. reducing with hydrogen in the presence of a suitable catalyst to form the corresponding amine;
d. forming the compound of formula : 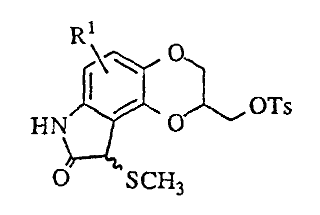 by reaction with ethyl methyl thioacetate in the presence of a solvent;
e. cleaving the thiomethyl ether by treatment with Raney nickel;
f. replacing the tosylate or halide with the appropriate azaheterocycle in the presence of a high boiling solvent to form the compound of formula (I); and optionally forming a pharmaceutically acceptable salt thereof.

24. Process for the preparation of compounds of formula I in which X is oxygen which comprises preparation of the compound by a process as defined in any of claims 21, 22, and 23 and at a suitable step in the process, oxidising the corresponding oxindole.

25. Process according to claim 21 in which steps a. and b. are replaced with either
A. alkylating an appropriately substituted 5- or 6-salicylaldehyde with an allyl halide to form the compound of formula: 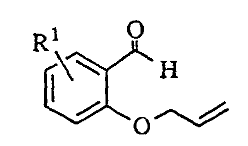 converting the aldehyde to a phenol by treatment with a peroxybenzoic acid followed by cleavage of the intermediate ester: 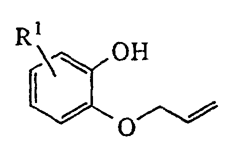 and reacting with nitric acid to form the compound of formula : 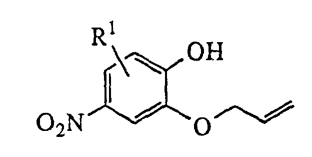 or
B. protecting the hydroxyl group on an appropriately substituted 3- or 4-salicylaldehyde with [2-(trimethylsilyl)ethoxy]methyl chloride (SEMCl) by forming the compound of formula: 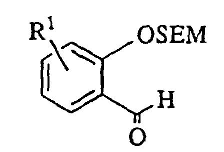 while converting the aldehyde to the hydroxyl by treatment with a perbenzoic acid followed by hydrolysis and then reacting with nitric acid to form the compound of formula: 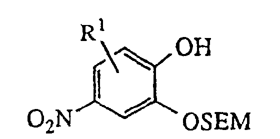 then alkylating with an allyl halide to form the compound of formula:

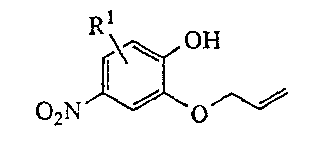

## Patentansprüche

1. Verbindung der Formel I 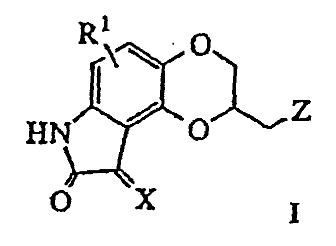 worin
X für H₂ oder O steht;
R¹ Wasserstoff, Hydroxy, Halogen, Trifluormethyl, Trifluormethoxy, Alkyl mit 1 bis 6 Kohlenstoffatomen, Alkoxy mit 1 bis 6 Kohlenstoffatomen, Aralkoxy mit 7 bis 12 Kohlenstoffatomen, Alkanoyioxy mit 2 bis 6 Kohlenstoffatomen, Aminosäure, Mono- oder Di-alkylamino, worin jede Alkylgruppe 1 bis 6 Kohlenstoffatome hat, Alkanamido mit 2 bis 6 Kohlenstoffatomen oder Alkansulfonamido mit 1 bis 6 Kohlenstoffatomen darstellt;
Z definiert ist durch 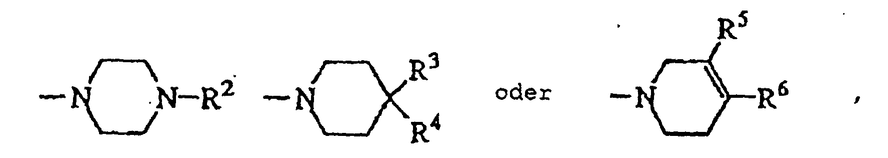
worin
R² Wasserstoff, Alkyl mit 1 bis 6 Kohlenstoffatomen, Cycloalkyl mit 3 bis 8 Kohlenstoffatomen, Phenyl, gegebenenfalls substituiert mit R¹ wie oben definiert; ω-Phenylalkyl oder ω-Diphenylalkyl darstellt, worin die Alkylkette 1 bis 4 Kohlenstoff-atome enthält und der Phenylring gegebenenfalls substituiert ist mit R¹ wie oben definiert, oder R² für Naphthyl, Indolyl, Indazolyl, Thienyl, Pyridinyl, Pyrimidinyl, Chinolinyl, Benzoiso-thiazolyl oder Benzisoxazolyl steht, jedes gegebenenfalls substituiert mit R¹ wie oben definiert;
R³ Wasserstoff darstellt, und R⁴ Wasserstoff, 1-Benzimidazolyl-2-on, Indolyl, Benzoisothiazolyl oder Benzisoxazol darstellt, jedes gegebenenfalls substituiert mit R¹ wie oben definiert, oder R⁴ für -Y-Ar steht, worin Y für C=O, CHOH oder (CH₂)ₘ steht, worin m 0 bis 4 ist und Ar Phenyl darstellt, gegebenenfalls substituiert mit R¹ wie oben definiert,
oder R³ und R⁴ zusammengenommen mit dem Kohlenstoffatom, an welches sie gebunden sind 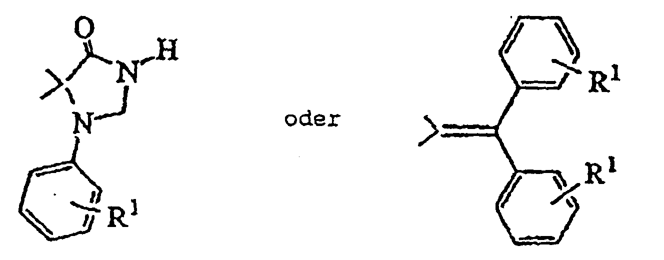 bilden,
R⁵ Wasserstoff darstellt und R⁶ Phenyl, Naphthyl, Thienyl, Indolyl, Benzoisothiazolyl oder Benzisoxazolyl darstellt, jedes gegebenenfalls substituiert mit R¹ wie oben definiert,
oder R⁵ und R⁶, zusammengenommen mit den Kohlenstoffatomen, an welche sie gebunden sind, einen Benzolring vervollständigen, gegebenenfalls substituiert mit R¹;
oder ein pharmazeutisch annehmbares Salz davon.

2. Verbindung nach Anspruch 1, worin R² Phenyl, Naphthyl, Indolyl, Indazolyl, Thienyl, Pyridinyl, Pyrimidinyl, Chinolinyl, Benzoisothiazolyl oder Benzisoxazolyl darstellt, jedes gegebenenfalls substituiert mit R¹ wie in Anspruch 1 definiert, R³ Wasserstoff darstellt und R⁴ 1-Benzimidazolyl-2-on, Indolyl, Benzoisothiazolyl oder Benzisoxazolyl darstellt, jedes gegebenenfalls substituiert mit R¹ wie in Anspruch 1 definiert, oder R⁴ für -Y-Ar steht, worin Y für C=O steht und Ar Phenyl darstellt, gegebenenfalls substituiert mit R¹ wie in Anspruch 1 definiert, R⁵ Wasserstoff darstellt und R⁶ Phenyl, Indolyl, Benzoisothiazolyl oder Benzisoxazolyl darstellt, jedes gegebenenfalls substituiert mit R¹ wie in Anspruch 1 definiert, oder R⁵ und R⁶ zusammengenommen mit den Kohlenstoffatomen, an welche sie gebunden sind, einen Benzolring vervollständigen, gegebenenfalls substituiert mit R¹;
oder ein pharmazeutisch annehmbares Salz davon.

3. Verbindung gemäß Anspruch 1, worin X für H₂ steht, R² Phenyl, Indolyl, Pyridinyl, Pyrimidinyl, Chinolinyl, Benzoisothiazolyl oder Benzisoxazolyl darstellt, jedes gegebenenfalls substituiert mit R¹ wie in Anspruch 1 definiert, R⁵ Wasserstoff darstellt und R⁶ Phenyl darstellt, gegebenenfalls substituiert mit R¹ wie oben definiert, oder R⁵ undR⁶ zusammen mit den Kohlenstoffatomen, wen welche sie gebunden sind, einen Benzolring vervollständigen, gegebenenfalls substituiert mit R¹; oder ein pharmazeutisch annehmbares Salz davon.

4. Verbindung nach Anspruch 1, welche 2-(3,4-Dihydro-1Hisochinolin-2-ylmethyl)-2,3,8,9-tetrahydro-7H-1,4-dioxino[2,3-e]-indol-8-on ist, oder ein pharmazeutisch annehmbares Salz davon.

5. Verbindung nach Anspruch 1, welche 2-[4-(4-Fluor-benzoyl)-piperidin-1-ylmethyl]-2,3,8,9-tetrahydro-7H-1,4-dioxino[2,3-e]-indol-8-on ist, oder ein pharmazeutisch annehmbares Salz davon.

6. Verbindung nach Anspruch 1, welche 2-(4-Oxo-l-phenyl-1,3,8-triazo-spiro[4,5]dec-8-ylmethyl)-2,3,8,9-tetrahydro-7H-1,4-dioxino[2,3-e]indol-8-on ist, oder ein pharmazeutisch annehmbares Salz davon.

7. Verbindung nach Anspruch 1, welche 2-[4-(2-Oxo-2,3-dihydrobenzimidazol-1-yl)-piperidin-1-ylmethyl]-2,3,8,9-tetrahydro-7H-1,4-dioxino[2,3-e]indol-8-on ist, oder ein pharmazeutisch annehmbares Salz davon.

8. Verbindung nach Anspruch 1, welche 2-(4-Phenyl-3,6-dihydro-2H-pyridin-1-ylmethyl)-2,3,8,9-tetrahydro-7H-1,4-dioxino[2,3-e]-indol-8-on ist, oder ein pharmazeutisch annehmbares Salz davon.

9. Verbindung nach Anspruch 1, welche 2-[4-(1H-Indol-4-yl)-piperazin-1-ylmethyl]-2,3,8,9-tetrahydro-7H-1,4-dioxino[2,3-e]indol-8-on ist, oder ein pharmazeutisch annehmbares Salz davon.

10. Verbindung nach Anspruch 1, welche 2-(3,4-Dihydro-1Hisochinolin-2-ylmethyl)-6-fluor-2,3,8,9-tetrahydro-7H-1,4-dioxino-[2,3-e]indol-8-on ist, oder ein pharmazeutisch annehmbares Salz davon.

11. Verbindung nach Anspruch 1, welche 2-[4-(1H-Indol-3-yl)-1-piperidinylmethyl]-6-fluor-2,3,8,9-tetrahydro-7H-1,4-dioxino-[2,3-e]indol-8-on ist, oder ein pharmazeutisch annehmbares Salz davon.

12. Verbindung nach Anspruch 1, welche 2-[4-(1,2-Benzisothiazol-3-yl)-1-piperazinylmethyl)-2,3,8,9-tetrahydro-7H-1,4-dioxino-[2,3-e]indol-8-on ist, oder ein pharmazeutisch annehmbares Salz davon.

13. Verbindung nach Anspruch 1, welche 2-[4-(6-Fluor-1,2-benzisoxazol-3-yl)-1-piperidinylmethyl]-2,3,8,9-tetrahydro-7H-1,4-dioxino[2,3-e]indol-8-on ist, oder ein pharmazeutisch annehmbares Salz davon.

14. Verbindung nach Anspruch 1, welche 4-Fluor-8-[4-(1H-indol-3-yl)-3,6-dihydro-2H-pyridin-1-ylmethyl]-1,3,7,8-tetrahydro-6,9-dioxa-3-aza-cyclopenta[a]naphthalin-2-on ist, oder ein pharmazeutisch annehmbares Salz davon.

15. Verwendung einer Verbindung der Formel I, wie in einem der Ansprüche 1 bis 14 definiert, zur Herstellung eines Arzneimittels zur Behandlung von Erkrankungen von Dopamin-Fehlsteuerung des Gehirns, welche Behandlung Verabreichen des Arzneimittels oral oder parenteral an eine Person umfasst, welche an solch einer Störung des dopaminergen Systems leidet.

16. Verwendung einer Verbindung gemäß Anspruch 15 zur Behandlung von Schizophrenie oder einer schizoaffektiven Störung, welche Verabreichen einer Menge einer Verbindung der Formel I oral oder parenteral an eine Person umfasst, welche an Fehlsteuerung des dopaminergen Systems leidet.

17. Pharmazeutische Zusammensetzung, welche eine Verbindung der Formel (I), wie in einem der Ansprüche 1 bis 14 definiert, und einen pharmazeutisch annehmbaren Träger dafür umfasst.

18. Verbindung wie in einem der Ansprüche 1 bis 14 definiert, zur Verwendung als Pharmazeutikum oder therapeutische Substanz.

19. Verbindung wie in einem der Ansprüche 1 bis 14 definiert, zur Verwendung bei der Behandlung von Erkrankungen von Dopamin-Fehlsteuerung des Gehirns.

20. Verbindung gemäß Anspruch 19 zur Verwendung bei der Behandlung von Schizophrenie oder einer schizoaffektiven Störung.

21. Verfahren zur Herstellung einer Verbindung der Formel I wie in einem der Ansprüche 1 bis 14 definiert, welches umfasst:
a. Alkylieren eines passend substituierten Nitroguajacol mit einem Allylhalogenid;
b. Demethylieren der sich ergebenden Verbindung, um die Verbindung der Formel: 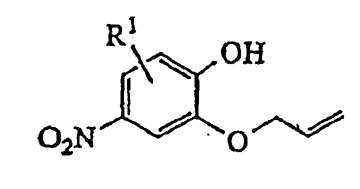 zu bilden;
c. Alkylieren mit einem Glycidyltosylat oder einem Epihalogenhydrin in Gegenwart einer Base, um die Verbindung der Formel: 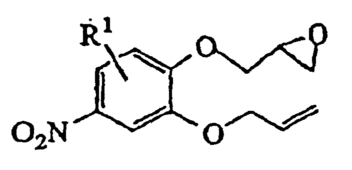 zu bilden;
d. Erhitzen der sich ergebenden Verbindung in einem hochsiedenden Lösungsmittel, um die Verbindung der Formel: 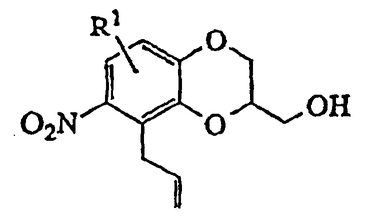 zu bilden;
e. Umwandeln in ein Tosylat durch Umsetzung mit p-Toluolsulfonylchlorid in Gegenwart von Pyridin oder alternierend in ein Halogenid durch Umsetzung mit Tetrabromkohlenstoff oder Tetrachlorkohlenstoff in Verbindung mit Triphenylphosphin;
f. Umwandeln der Allyl-Seitenkette in eine Essigsäurekomponente durch oxidative Spaltung;
g. Reduzieren der Nitrogruppe zu einem Amin;
h. Cyclisieren des Amin und der Säuregruppe zu Lactam unter Verwendung einer verdünnten Säure;
i. Ersetzen des Tosylat oder Halogenid mit dem passenden Azaheterocyclus in Gegenwart eines hochsiedenden Lösungsmittels, um die Verbindung der Formel (I) zu bilden; und gegebenenfalls Bilden eines pharmazeutisch annehmbaren Salzes davon.

22. Verfahren zur Herstellung von Verbindungen der Formel I wie in einem der Ansprüche 1 bis 14 definiert, welches umfasst:
a. Alkylieren eines passend substituierten Salicylaldehyd mit einem Allylhalogenid, um die Verbindung der Formel: 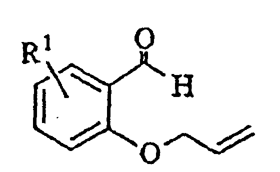 zu bilden;
b. Umwandeln des Aldehyd in ein Phenol durch Behandlung mit einer Peroxybenzoesäure, gefolgt von Spaltung des Ester-Zwischenverbindung: 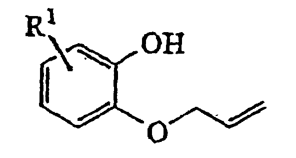
c. Alkylieren mit Glycidyltosylat oder einem Epihalogenhydrin in Gegenwart einer Base, um die Verbindung der Formel: 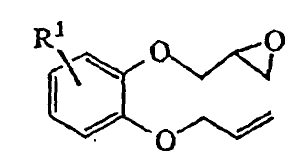 zu bilden;
d. Erhitzen in einem hochsiedenden Lösungsmittel gefolgt von Cyclisierung, um die Verbindung der Formel 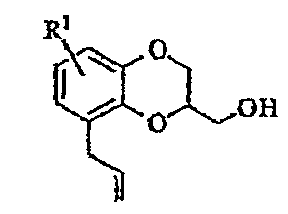 zu bilden;
e. Umwandeln in ein Tosylat durch Umsetzen mit p-Toluolsulfonylchlorid in Gegenwart von Pyridin oder alternierend in ein Halogenid durch Umsetzung mit Tetrabromkohlenstoff oder Tetrachlorkohlenstoff in Verbindung mit Triphenylphosphin;
f. Umwandeln der Allyl-Seitenkette in eine Essigsäuregruppe durch oxidative Spaltung;
g. Einführen einer Nitrogruppe durch Behandlung mit Salpetersäure in einem Lösungsmittel;
h. Reduzieren der Nitrogrupe zu einem Amin;
i. Cyclisieren des Amin und der Säuregruppe in das entsprechende Lactam;
j. Ersetzen des Tosylat oder Halogenid mit dem passenden Azaheterocyclus in Gegenwart eines hochsiedenden Lösungsmittels, um die Verbindung der Formel I zu bilden; und gegebenenfalls bilden eines pharmazeutisch annehmbaren Salzes davon.

23. Verfahren zur Herstellung einer Verbindung der Formel I wie in einem der Ansprüche 1 bis 14 definiert, welches umfasst:
a. Umwandeln eines passend substituierten Benzodioxanmethanol der Formel: 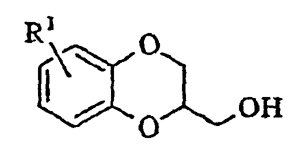 in ein Methyltosylat durch Umsetzung mit p-Toluolsulfonylchlorid in Gegenwart von Pyridin;
b. Umsetzen der sich ergebenden Verbindung mit Salpetersäure in einem Lösungsmittel, um die entsprechende Nitro-substituierte Verbindung der Formel: 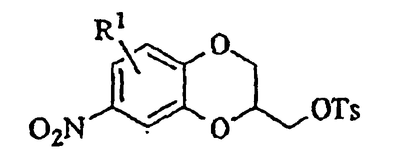 zu erhalten;
c. Reduzieren mit Wasserstoff in Gegenwart eines geeigneten Katalysators, um das entsprechende Amin zu bilden;
d. Bilden der Verbindung der Formel: 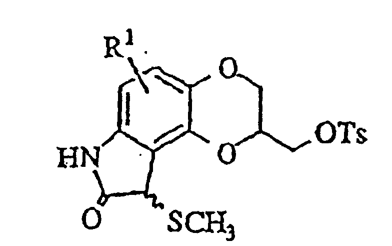 durch Umsetzung mit Ethylmethylthioacetat in Gegenwart eines Lösungsmittels;
e. Spalten des Thiomethylethers durch Behandlung mit Raney-Nickel;
f. Ersetzen des Tosylat oder Halogenid mit dem passenden Azaheterocyclus in Gegenwart eines hochsiedenden Lösungsmittels, um die Verbindung der Formel (I) zu bilden; und gegebenenfalls Bilden eines pharmazeutisch annehmbaren Salzes davon.

24. Verfahren zur Herstellung von Verbindungen der Formel I worin X für Sauerstoff steht, welches umfasst: Herstellung der Verbindung durch ein Verfahren, wie in einem der Ansprüche 21, 22 und 23 definiert, und bei einem geeigneten Schritt in dem Verfahren, Oxidieren des entsprechenden Oxindol.

25. Verfahren gemäß Anspruch 21, worin Schritt a. und b. ersetzt werden mit entweder:
A. Alkylieren eines passend substituierten 5- oder 6-Salicylaldehyd mit einem Allylhalogenid, um die Verbindung der Formel 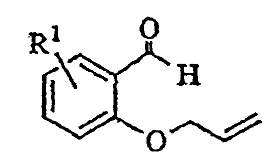 zu bilden,
Umwandeln des Aldehyd in ein Phenol durch Behandlung mit einer Peroxybenzoesäure, gefolgt von Spaltung der Esterzwischenverbindung: 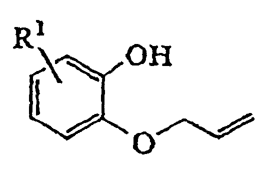 und Umsetzen mit Salpetersäure, um die Verbindung der Formel: 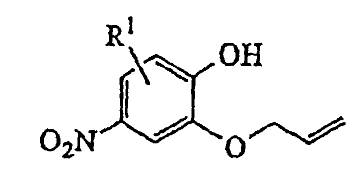 zu bilden; oder
B. Schützen der Hydroxylgruppe an einem passend substituierten 3- oder 4-Salicylaldehyd mit [2-Trimethylsilyl)ethoxy]methylchlorid (SEMCl) durch Bilden der Verbindung der Formel: 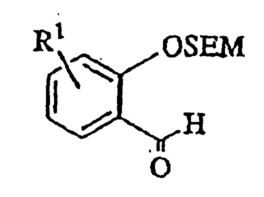 während das Aldehyd in das Hydroxyl umgewandelt wird durch Behandlung mit einer Perbenzoesäure, gefolgt von Hydrolyse, und dann Umsetzen mit Salpetersäure, um die Verbindung der Formel: 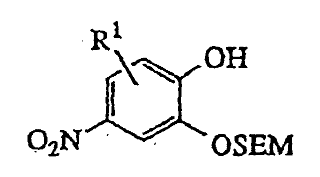 zu bilden; dann Alkylieren mit einem Allylhalogenid, um die Verbindung der Formel: 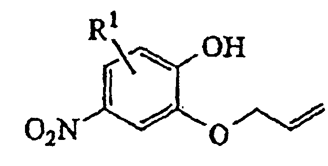 zu bilden.

## Revendications

1. Composé de formule I 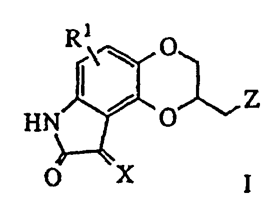 dans laquelle
X est H₂ ou O;
R¹ est un hydrogène, un hydroxy, un halogéno, un trifluorométhyle, un trifluorométhoxy, un alkyle de 1 à 6 atomes de carbone, un alcoxy de 1 à 6 atomes de carbone, un aralcoxy de 7 à 12 atomes de carbone, un alcanoyloxy de 2 à 6 atomes de carbone, un amino, un mono- ou un dialkylamino dans lequel chaque groupement alkyle présente 1 à 6 atomes de carbone, un alcanamido de 2 à 6 atomes de carbone, ou un alcanesulfonamido de 1 à 6 atomes de carbone;
Z est défini par 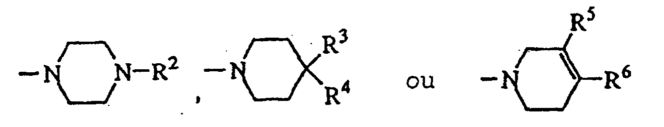
dans lesquelles
R² est un hydrogène, un alkyle de 1 à 6 atomes de carbone, un cycloalkyle de 3 à 8 atomes de carbone, un phényle facultativement substitué par R¹ comme défini ci-dessus; un ω-phénylalkyle ou ω-diphénylalkyle, dans lequel la chaîne alkyle contient 1 à 4 atomes de carbone et le cycle phényle est facultativement substitué par R¹ comme défini ci-dessus, ou R² est un naphtyle, un indolyle, un indazolyle, un thiényle, un pyridinyle, un pyrimidinyle, un quinolinyle, un benzoisothiazolyle ou un benzisoxazolyle, chacun étant facultativement substitué par R¹ comme défini ci-dessus;
R³ est un hydrogène et R⁴ est un hydrogène, une 1-benzimidazolyl-2-one, un indolyle, un benzoisothiazolyle ou un benzisoxazole, chacun étant facultativement substitué par R¹ comme défini ci-dessus, ou R⁴ est -Y-Ar, dans laquelle Y est C=O, CHOH, ou (CH₂)ₘ, dans laquelle m est 0 à 4, et Ar est un phényle facultativement substitué par R¹ comme défini ci-dessus,
ou R³ et R⁴, pris ensemble avec l'atome de carbone auquel ils sont liés forment 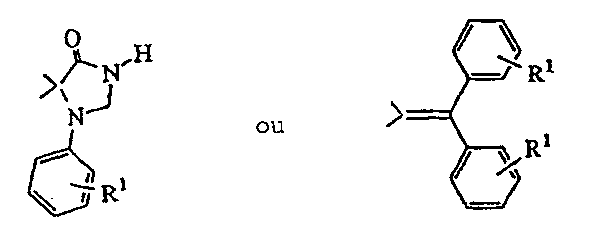
R⁵ est un hydrogène et R⁶ est un phényle, un naphtyle, un thiényle, un indolyle, un benzoisothiazolyle ou un benzisoxazolyle, chacun étant facultativement substitué par R¹ comme défini ci-dessus,
ou R⁵ et R⁶, pris ensemble avec les atomes de carbone auxquels ils sont liés forment un cycle benzène facultativement substitué par R¹;
ou un sel pharmaceutiquement acceptable de celui-ci.

2. Composé suivant la revendication 1, dans lequel R² est un phényle, un naphtyle, un indolyle, un indazolyle, un thiényle, un pyridinyle, un pyrimidinyle, un quinolinyle, un benzoisothiazolyle, ou un benzisoxazolyle, chacun étant facultativement substitué par R¹ comme défini à la revendication 1, R³ est un hydrogène, et R⁴ est une 1-benzimidazolyl-2-one, un indolyle, un benzoisothiazolyle, ou un benzisoxazolyle, chacun étant facultativement substitué par R¹ comme défini à la revendication 1, ou R⁴ est -Y-Ar, dans laquelle Y est C=O, et Ar est un phényle facultativement substitué par R¹ comme défini à la revendication 1, R⁵ est un hydrogène et R⁶ est un phényle, un indolyle, un benzoisothiazolyle ou un benzisoxazolyle, chacun étant facultativement substitué par R¹ comme défini à la revendication 1, ou R⁵ et R⁶, pris ensemble avec les atomes de carbone auxquels ils sont liés forment un cycle benzène facultativement substitué par R¹; ou un sel pharmaceutiquement acceptable de celui-ci.

3. Composé suivant la revendication 1, dans lequel X est H₂, R² est un phényle, un indolyle, un pyridinyle, un pyrimidinyle, un quinolinyle, un benzoisothiazolyle ou un benzisoxazolyle, chacun étant facultativement substitué par R¹ comme défini suivant la revendication 1, R⁵ et un hydrogène et R⁶ est un phényle, facultativement substitué par R¹ comme défini ci-dessus,
ou R⁵ et R⁶, pris ensemble avec les atomes de carbone auxquels ils sont liés forment un cycle benzène facultativement substitué par R¹; ou un sel pharmaceutiquement acceptable de celui-ci.

4. Composé suivant la revendication 1, qui est la 2-(3,4-dihydro-1H-isoquinolin-2-ylméthyl)-2,3,8,9-tétrahydro-7H-1,4-dioxino[2,3-e]indol-8-one ou un sel pharmaceutiquement acceptable de celle-ci.

5. Composé suivant la revendication 1, qui est la 2-[4-(4-fluorobenzoyl)pipéridin-1-ylméthyl]-2,3,8,9-tétrahydro-7H-1,4-dioxino[2,3-e]indol-8-one ou un sel pharmaceutiquement acceptable de celle-ci.

6. Composé suivant la revendication 1, qui est la 2-(4-oxo-1-phényl-1,3,8-triazaspiro[4,5]déc-8-yl-méthyl)-2,3,8,9-tétrahydro-7H-1,4-dioxino[2,3-e]indol-8-one ou un sel pharmaceutiquement acceptable de celle-ci.

7. Composé suivant la revendication 1, qui est la 2-[4-(2-oxo-2,3-dihydrobenzimidazol-1-yl)-pipéridin-1-ylméthyl]-2,3,8,9-tétrahydro-7H-1,4-dioxino-[2,3-e]indol-8-one ou un sel pharmaceutiquement acceptable de celle-ci.

8. Composé suivant la revendication 1, qui est la 2-(4-phényl-3,6-dihydro-2H-pyridin-1-ylméthyl)-2,3,8,9-tétrahydro-7H-1,4-dioxino[2,3-e]indol-8-one ou un sel pharmaceutiquement acceptable de celle-ci.

9. Composé suivant la revendication 1, qui est la 2-[4-(1H-indol-4-yl)pipérazin-l-ylméthyl]-2,3,8,9-tétrahydro-7H-1,4-dioxino[2,3-e]indol-8-one ou un sel pharmaceutiquement acceptable de celle-ci.

10. Composé suivant la revendication 1, qui est la 2-(3,4-dihydro-1H-isoquinolin-2-ylméthyl)-6-fluoro-2,3,8,9-tétrahydro-7H-1,4-dioxino[2,3-e]indol-8-one ou un sel pharmaceutiquement acceptable de celle-ci.

11. Composé suivant la revendication 1, qui est la 2-[4-(1H-indol-3-yl)-1-pipéridinylméthyl]-6-fluoro-2,3,8,9-tétrahydro-7H-1,4-dioxino[2,3-e]indol-8-one ou un sel pharmaceutiquement acceptable de celle-ci.

12. Composé suivant la revendication 1, qui est la 2-[4-(1,2-benzisothiazol-3-yl)-1-pipérazinyl-méthyl]-2,3,8,9-tétrahydro-7H-1,4-dioxino[2,3-e]indol-8-one ou un sel pharmaceutiquement acceptable de celle-ci.

13. Composé suivant la revendication 1, qui est la 2-[4-(6-fluoro-1,2-benzisoxazol-3-yl)-1-pipéridinylméthyl]-2,3,8,9-tétrahydro-7H-1,4-dioxino-[2,3-e]indol-8-one ou un sel pharmaceutiquement acceptable de celle-ci.

14. Composé suivant la revendication 1, qui est la 4-fluoro-8-[4-(1H-indol-3-yl)-3,6-dihydro-2H-pyridin-1-ylméthyl]-1,3,7,8-tétrahydro-6,9-dioxa-3-azacyclopenta[a]naphtalèn-2-one ou un sel pharmaceutiquement acceptable de celle-ci.

15. Utilisation d'un composé de formule I, suivant l'une quelconque des revendications 1 à 14, pour la préparation d'un médicament pour le traitement de maladies de dysfonctionnement de la régulation de la dopamine dans le cerveau, lequel traitement comprend l'administration du médicament, par voie orale ou parentérale à un sujet souffrant d'un trouble de-ce type du système dopaminergique.

16. Utilisation d'un composé suivant la revendication 15, pour le traitement d'une schizophrénie ou d'une schizophrénie dysthymique, qui comprend l'administration par voie orale ou parentérale à un sujet souffrant d'un dysfonctionnement de la régulation du système dopaminergique, d'une quantité d'un composé de formule I.

17. Composition pharmaceutique comprenant un composé de formule (I) suivant l'une quelconque des revendications 1 à 14, et un vecteur pharmaceutiquement acceptable pour celle-ci.

18. Composé suivant l'une quelconque des revendications 1 à 14, pour une utilisation comme substance pharmaceutique ou thérapeutique.

19. Composé suivant l'une quelconque des revendications 1 à 14, pour une utilisation dans le traitement de maladies de dysfonctionnement de la régulation de la dopamine dans le cerveau.

20. Composé suivant la revendication 19, pour une utilisation dans le traitement d'une schizophrénie ou d'une schizophrénie dysthymique.

21. Procédé pour la préparation de composés de formule I, suivant l'une quelconque des revendications 1 à 14 comprenant :
a. l'alkylation d'un nitroguaiacol substitué de manière appropriée avec un halogénure d'allyle;
b. la déméthylation du composé obtenu pour former le composé de formule : 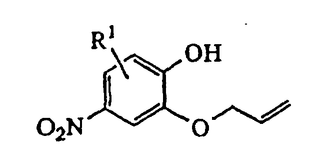
c. l'alkylation avec du tosylate de glycidyle ou une épihalogénohydrine en présence d'une base pour former le composé de formule : 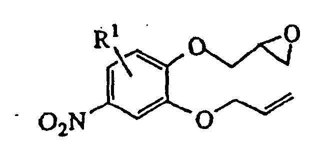
d. le chauffage du composé obtenu dans un solvant de haut point d'ébullition pour former le composé de formule : 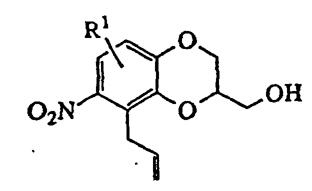
e. la conversion en un tosylate par réaction avec du chlorure de p-toluènesulfonyle en présence de pyridine ou en variante en un halogénure par réaction avec du tétrabromure de carbone ou du tétrachlorure de carbone en combinaison avec de la triphénylphosphine;
f. la conversion de la chaîne latérale allyle en une partie acide acétique par un clivage oxydatif;
g. la réduction du groupement nitro en une amine;
h. la cyclisation de l'amine et du groupement acide en un lactame en utilisant un acide dilué;
i. le remplacement du tosylate ou de l'halogénure par l'azahétérocycle approprié en présence d'un solvant de haut point d'ébullition pour former le composé de formule (I); et facultativement la formation d'un sel pharmaceutiquement acceptable de celui-ci.

22. Procédé de préparation des composés de formule I suivant l'une quelconque des revendications 1 à 14 comprenant :
a. l'alkylation d'un salicylaldéhyde substitué de manière appropriée avec un halogénure d'allyle pour former le composé de formule : 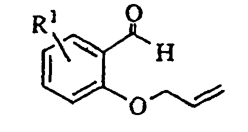
b. la conversion de l'aldéhyde en un phénol par un traitement avec un acide peroxybenzoïque suivi par le clivage de l'ester intermédiaire : 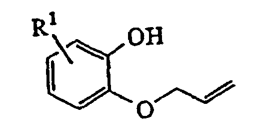
c. l'alkylation avec du tosylate de glycidyle ou une épihalogénohydrine en présence d'une base pour former le composé de formule : 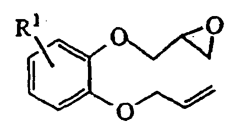
d. le chauffage dans un solvant de haut point d'ébullition suivi par une cyclisation pour former le composé de formule : 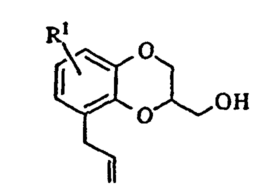
e. la conversion en un tosylate par réaction avec du chlorure de p-toluènesulfonyle en présence de pyridine ou en variante en un halogénure par réaction avec du tétrabromure de carbone ou du tétrachlorure de carbone en combinaison avec de la triphénylphosphine;
f. la conversion de la chaîne latérale allyle en un groupement acide acétique par un clivage oxydatif;
g. l'introduction d'un groupement nitro par traitement avec de l'acide nitrique dans un solvant;
h. la réduction du groupement nitro en une amine;
i. la cyclisation de l'amine et du groupement acide en le lactame correspondant;
j. le remplacement du tosylate ou de l'halogénure par l'azahétérocycle approprié en présence d'un solvant de haut point d'ébullition pour former le composé de formule I;
et facultativement la formation d'un sel pharmaceutiquement acceptable de celui-ci.

23. Procédé de préparation de composés de formule I suivant l'une quelconque des revendications 1 à 14, comprenant
a. la conversion d'un benzodioxanne méthanol substitué de manière appropriée de formule : 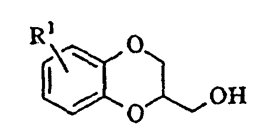 en un tosylate de méthyle par réaction avec du chlorure de p-toluènesulfonyle en présence de pyridine;
b. la réaction du composé obtenu avec de l'acide nitrique dans un solvant pour obtenir le composé à substitution nitro correspondant de formule : 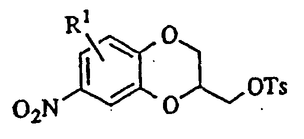
c. la réduction avec de l'hydrogène en présence d'un catalyseur approprié pour former l'amine correspondante;
d. la formation du composé de formule : 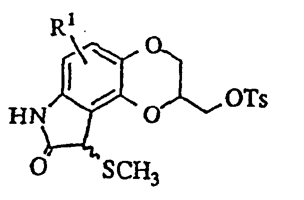 par réaction avec de l'éthylméthylthioacétate en présence d'un solvant;
e. le clivage du thiométhyléther par traitement avec du nickel de Raney;
f. le remplacement du tosylate ou de l'halogénure par l'azahétérocycle approprié en présence d'un solvant de haut point d'ébullition pour former le composé de formule (I) ; et facultativement la formation d'un sel pharmaceutiquement acceptable de celui-ci.

24. Procédé pour la préparation de composés de formule I dans lesquels X est un oxygène qui comprend la préparation du composé par un procédé suivant l'une quelconque des revendications 21, 22 et 23 et à une étape appropriée dans le procédé, l'oxydation de l'oxindole correspondant.

25. Procédé suivant la revendication 21, dans lequel les étapes a. et b. sont remplacés soit par
A. l'alkylation d'un salicylaldéhyde substitué de manière appropriée en 5 ou en 6 par un halogénure d'allyle pour former le composé de formule : 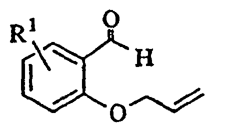 la conversion de l'aldéhyde en un phénol par traitement avec un acide peroxybenzoïque suivi par un clivage de l'ester intermédiaire : 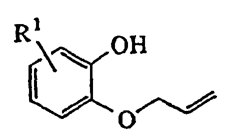 et la réaction avec de l'acide nitrique pour former le composé de formule : 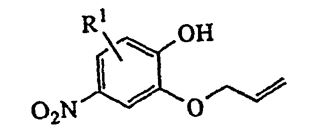 soit
B. la protection du groupement hydroxyle sur un salicylaldéhyde substitué de manière appropriée en 3 ou en 4 avec du chlorure de [2-(triméthylsilyl)éthoxy]-méthyle (SEMCI), ce qui forme le composé de formule : 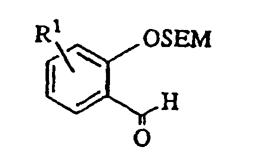 pendant la conversion de l'aldéhyde en l'hydroxyle par traitement avec un acide perbenzoïque suivie par une hydrolyse puis une réaction avec de l'acide nitrique pour former le composé de formule : 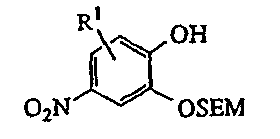 puis l'alkylation avec un halogénure d'allyle pour former le composé de formule :
